# EUROPEAN PATENT SPECIFICATION

(11) **EP 2 844 172 B1**
(45) Date of publication and mention of the grant of the patent: **04.10.2017**
(21) Application number: 13785129.1
(22) Date of filing: 01.05.2013
(51) Int. Cl.: A61B 18/12, A61B 18/14

(54) **ELECTROSURGICAL DEVICE FOR CUTTING AND COAGULATING**
ELEKTROCHIRURGISCHE SCHNEID- UND KOAGULATIONSVORRICHTUNG
DISPOSITIF ÉLECTRO-CHIRURGICAL POUR DÉCOUPER ET COAGULER

(30) Priority: 02.05.2012 US 201261641443 P
(43) Date of publication of application: 11.03.2015
(73) Proprietor: Ethicon LLC, Guaynabo, PR 00969 (US)
(72) Inventor: BOUDREAUX, Chad P., Cincinnati, OH 45242 (US); HUANG, Zhifan F., Mason, OH 45040 (US); MILLER, Matthew C., Cincinnati, OH 45212 (US); O'CONNOR, Megan A., West Chester, OH 45069 (US); PAPA, Christopher A., Cincinnati, OH 45241 (US); SCHULTE, John B., West Chester, OH 45069 (US); TIMM, Richard W., Cincinnati, OH 45209 (US); WITT, David A., Maineville, OH 45039 (US); ZINGMAN, Aron O., Cambridge, MA 02139 (US)
(74) Representative: Carpmaels & Ransford LLP
(86) International application number: PCT/US2013/039015
(87) International publication number: WO 2013/166115

(56) References cited:
- WO-A1-99/12488
- CN-U- 202 086 580
- US-A- 4 241 861
- US-A1- 2002 107 517
- US-A1- 2005 107 784
- US-A1- 2006 271 102
- US-A1- 2010 312 240
- US-A1- 2011 257 680
- US-B2- 7 169 146

## Description

### BACKGROUND

A variety of surgical instruments include one or more elements that transmit RF energy to tissue (e.g., to coagulate or seal the tissue). Some such instruments comprise a pair of jaws that open and close on tissue, with conductive tissue contact surfaces that are operable to weld tissue clamped between the jaws. In open surgical settings, some such instruments may be in the form of forceps having a scissor grip. Such an instrument is disclosed by US2011/257680.

In addition to having RF energy transmission elements, some surgical instruments also include a translating tissue cutting element. An example of such a device is the ENSEAL® Tissue Sealing Device by Ethicon Endo-Surgery, Inc., of Cincinnati, Ohio.

Further examples of such devices and related concepts are disclosed in US2002/107517 and further in U.S. Pat. No. 6,500,176 entitled "Electrosurgical Systems and Techniques for Sealing Tissue," issued December 31, 2002; U.S. Pat. No. 7,112,201 entitled "Electrosurgical Instrument and Method of Use," issued
September 26, 2006; U.S. Pat. No. 7,125,409, entitled "Electrosurgical Working End for Controlled Energy Delivery," issued October 24, 2006; U.S. Pat. No. 7,169,146 entitled "Electrosurgical Probe and Method of Use," issued January 30, 2007; U.S. Pat. No. 7,186,253, entitled "Electrosurgical Jaw Structure for Controlled Energy Delivery," issued March 6, 2007; U.S. Pat. No. 7,189,233, entitled "Electrosurgical Instrument," issued March 13, 2007; U.S. Pat. No. 7,220,951, entitled "Surgical Sealing Surfaces and Methods of Use," issued May 22, 2007; U.S. Pat. No. 7,309,849, entitled "Polymer Compositions Exhibiting a PTC Property and Methods of Fabrication," issued December 18, 2007; U.S. Pat. No. 7,311,709, entitled "Electrosurgical Instrument and Method of Use," issued December 25, 2007; U.S. Pat. No. 7,354,440, entitled "Electrosurgical Instrument and Method of Use," issued April 8, 2008; U.S. Pat. No. 7,381,209, entitled "Electrosurgical Instrument," issued June 3, 2008.

Additional examples of electrosurgical cutting instruments and related concepts are disclosed in U.S. Pub. No. 2011/0087218, entitled "Surgical Instrument Comprising First and Second Drive Systems Actuatable by a Common Trigger Mechanism," published April 14, 2011; U.S. Pub. No. 2012/0116379, entitled "Motor Driven Electrosurgical Device with Mechanical and Electrical Feedback," published May 10, 2012; U.S. Pub. No. 2012/0078243, entitled "Control Features for Articulating Surgical Device," published March 29, 2012; U.S. Pub. No. 2012/0078247, entitled "Articulation Joint Features for Articulating Surgical Device," published March 29, 2012; U.S. Patent App. No. 13/622,729, entitled "Surgical Instrument with Multi-Phase Trigger Bias," filed September 19, 2012; and U.S. Patent App. No. 13/622,735, entitled "Surgical Instrument with Contained Dual Helix Actuator Assembly," filed September 19, 2012.

Some versions of electrosurgical instruments that are operable to sever tissue may be selectively used in at least two modes. One such mode may include both severing tissue and coagulating tissue. Another such mode may include just coagulating tissue without also severing the tissue. Yet another mode may include the use of jaws to grasp and manipulate tissue without also coagulating and/or severing the tissue. When an instrument includes grasping jaws and tissue severing capabilities, the instrument may also include a feature that ensures closure of the jaws before the tissue is severed.

While several medical devices have been made and used, it is believed that no one prior to the inventors has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1A depicts a side elevational view of an exemplary electrosurgical forceps instrument in an open configuration;
FIG. 1B depicts a side elevational view of the instrument of FIG. 1A in a closed configuration, with a firing beam in a proximal position;
FIG. 1C depicts a side elevational view of the instrument of FIG. 1A in a closed configuration, with a firing beam in a distal position;
FIG. 2 depicts a partial perspective view showing a joint of a first arm of the instrument of FIG. 1A entering a slot of a second arm of the instrument of FIG. 1A;
FIG. 3 depicts a cross-sectional side view of the end effector of the forceps instrument of FIG. 1A, with the end effector in an open configuration;
FIG. 4 depicts a cross-sectional end view of the end effector of the forceps instrument of FIG. 1A, with the end effector in a closed configuration;
FIG. 5 depicts a side elevational view of an exemplary alternative electrosurgical forceps instrument, with a housing cover removed;
FIG. 6 depicts a perspective view of another exemplary alternative electrosurgical forceps instrument;
FIG. 7 depicts a cross-sectional side view of the instrument of FIG. 6;
FIG. 8 depicts a partial perspective view showing a joint of a first arm of the instrument of FIG. 6 entering a slot of a second arm of the instrument of FIG. 6, with a housing half of the second arm removed;
FIG. 9 depicts an exploded perspective view of components of the instrument of FIG. 6;
FIG. 10 depicts a cross-sectional side view of a trigger assembly of the instrument of FIG. 6;
FIG. 11 depicts an exploded perspective view of the trigger assembly of FIG. 10;
FIG. 12A depicts a side elevational view of the trigger assembly of FIG. 10 in an unfired position, with a housing half of the second arm of the instrument removed;
FIG. 12B depicts a side elevational view of the trigger assembly of FIG. 10 in a fired position, with a housing half of the second arm of the instrument removed;
FIG. 13A depicts a cross-sectional side view of the instrument of FIG. 6 at an exemplary first instant of operation;
FIG. 13B depicts a cross-sectional side view of the instrument of FIG. 6 at an exemplary second instant of operation;
FIG. 13C depicts a cross-sectional side view of the instrument of FIG. 6 at an exemplary third instant of operation;
FIG. 13D depicts a cross-sectional side view of the instrument of FIG. 6 at an exemplary fourth instant of operation;
FIG. 14 depicts an exploded side view of an exemplary alternative electrosurgical forceps instrument;
FIG. 15 depicts a side elevational view of another exemplary alternative electrosurgical forceps instrument, with a disposable portion separated from a reusable portion;
FIG. 16 depicts a side elevational view of the instrument of FIG. 15, with the disposable portion coupled with the reusable portion;
FIG. 17 depicts a partial cross-sectional side view of the instrument of FIG. 15, showing the coupling between the disposable portion and the reusable portion;
FIG. 18 depicts a partial perspective view of the underside of the instrument of FIG. 15, showing the coupling between the disposable portion and the reusable portion;
FIG. 19 depicts a perspective view of another exemplary alternative electrosurgical instrument;
FIG. 20 depicts a side elevational view of the instrument of FIG. 19, with a disposable portion separated from the reusable portion;
FIG. 21 depicts a partial exploded view of the instrument of FIG. 19, with portions of the instrument omitted;
FIG. 22 depicts an exploded perspective view of the reusable portion of the instrument of FIG. 19;
FIG. 23 depicts another exploded perspective view of the reusable portion of the instrument of FIG. 19;
FIG. 24 depicts an exploded perspective view of the firing beam and firing beam locking member of the instrument of FIG. 19;
FIG. 25A depicts a partial side elevational view of the instrument of FIG. 19, showing the trigger in an unfired position, with a portion of the trigger housing omitted;
FIG. 25B depicts a partial side elevational view of the instrument of FIG. 19, showing the trigger in a fired position, with a portion of the trigger housing omitted;
FIG. 26 depicts a partial side elevational view of the instrument of FIG. 19, with a housing portion of the reusable portion omitted;
FIG. 27 depicts a perspective view of another exemplary alternative electrosurgical instrument;
FIG. 28 depicts a perspective view of the instrument of FIG. 27, with a disposable portion separated from a reusable portion;
FIG. 29 depicts an exploded perspective view of the disposable portion of the instrument of FIG. 27;
FIG. 30 depicts a partial top plan view of the disposable portion of the instrument of FIG. 27;
FIG. 31 depicts a side elevational view of the instrument of FIG. 27, with a housing portion of the reusable portion omitted;
FIG. 32 depicts a perspective view of a portion of the trigger of the instrument of FIG. 27;
FIG. 33 depicts a partial side elevational view of the instrument of FIG. 27, with a portion of the trigger housing omitted;
FIG. 34 depicts a partial side elevational view of the instrument of FIG. 27, with a with a housing portion of the reusable portion omitted and with a portion of the trigger housing omitted;
FIG. 35 depicts a perspective view of another exemplary alternative electrosurgical forceps instrument;
FIG. 36 depicts an exploded perspective view of the instrument of FIG. 35;
FIG. 37A depicts a partial cross-sectional side view of the instrument of FIG. 35, showing the instrument in an open configuration;
FIG. 37B depicts a partial cross-sectional side view of the instrument of FIG. 35, showing the instrument in a closed configuration;
FIG. 38 depicts a side elevational view of another exemplary alternative electrosurgical forceps instrument;
FIG. 39A depicts a cross-sectional side view of the instrument of FIG. 38, with a slider in a proximal position and a first arm in an open position;
FIG. 39B depicts a cross-sectional side view of the instrument of FIG. 38, with the slider in the proximal position and the first arm in a closed position;
FIG. 39C depicts a cross-sectional side view of the instrument of FIG. 38, with the slider in a first distal position and the first arm in the closed position;
FIG. 39D depicts a cross-sectional side view of the instrument of FIG. 38, with the slider in a second distal position and the first arm in the closed position;
FIG. 39E depicts a cross-sectional side view of the instrument of FIG. 38, with the slider in a third distal position and the first arm in the closed position; and
FIG. 39F depicts a cross-sectional side view of the instrument of FIG. 38, with the slider returned to the proximal position and the first arm in the closed position.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The invention is defined by the apparatus of independent claim 1. Preferred embodiments are defined by the dependent claims. Any disclosed methods are merely exemplary. The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument.

### I. Exemplary Electrosurgical Forceps

As previously noted, an electrosurgical instrument may include a set of jaws, with at least one of the jaws being pivotable relative to the other jaw to selectively compress
tissue between the jaws. Once the tissue is compressed, electrodes in the jaws may be activated with bipolar RF energy to seal the tissue. In some instances, a cutting feature is operable to sever tissue that is clamped between the jaws. For instance, the cutting feature may be actuated after the RF energy has sealed the tissue. Various references that are cited herein relate to electrosurgical instruments where the jaws are part of an end effector at the distal end of an elongate shaft, such that the end effector and the shaft may be inserted through a port (e.g., a trocar) to reach a site within a patient during a minimally invasive endoscopic surgical procedure. A handpiece may be positioned at the proximal end of the shaft for manipulating the end effector. Such a handpiece may have a pistol grip configuration or some other configuration.

In some instances, it may be desirable to provide an electrosurgical instrument that does not have an elongate shaft or handpiece similar to those described in the various references cited herein. In particular, it may be desirable to provide an electrosurgical instrument that is configured similar to a forceps device, with a scissor grip. Such instruments may be used in a variety of medical procedures. Various examples of electrosurgical shears/forceps devices are disclosed in U.S. Patent Application No. 13/752,588, entitled "Electrosurgical Hand Shears," filed January 29. 2013. Various other examples of electrosurgical forceps instruments will be described in greater detail below; while other examples will be apparent to those of ordinary skill in the art in view of the teachings herein.

### A. Exemplary Electrosurgical Forceps with Link-Driven Firing Beam

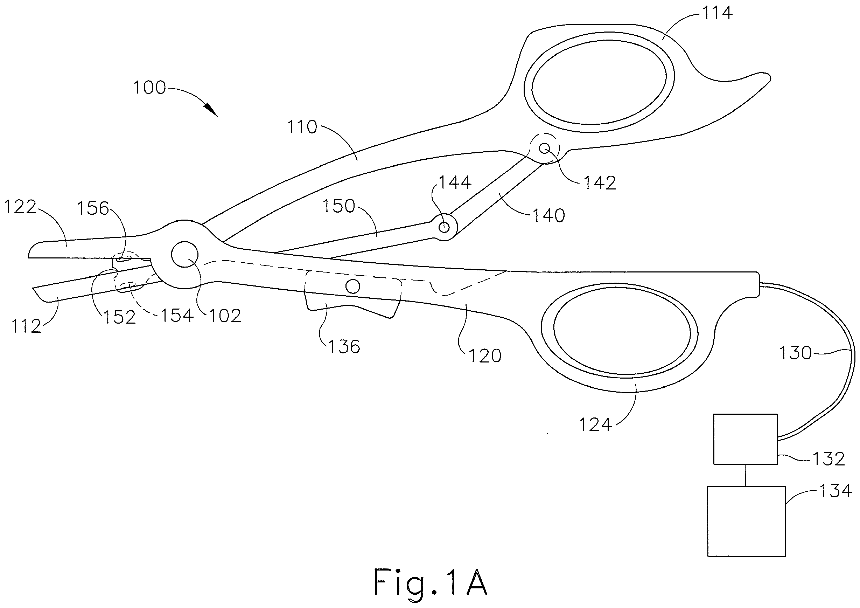

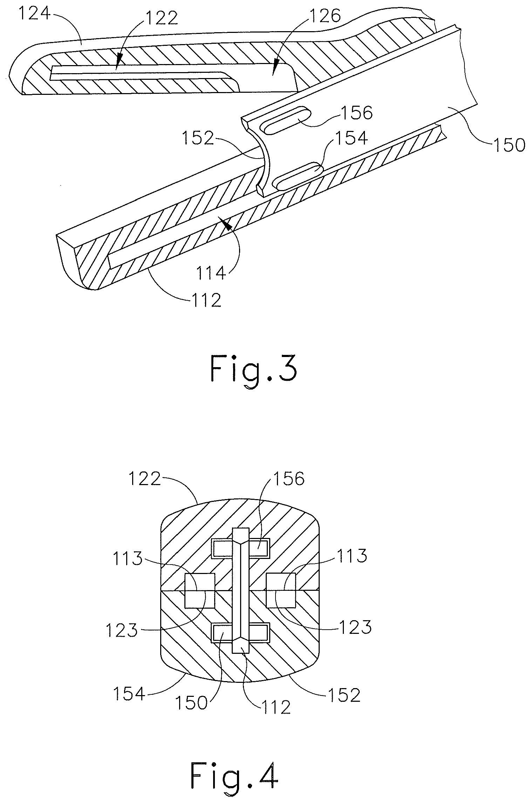

FIGS. 1A-1C show an exemplary electrosurgical forceps instrument (100) that may be used in three modes of operation, including grasping tissue, sealing tissue, and severing tissue. Instrument (100) of this example includes a first arm (110) and a second arm (120) that are pivotally coupled by a pin (102). A first jaw (112) is positioned at the distal end of first arm (110); while a thumb ring (114) is positioned at the proximal end of first arm (110). As shown in FIG. 4, first jaw (112) includes an electrode (113). Electrode (113) is U-shaped in the present example, with the bend of the U-shape located near the distal end of first jaw (112), such that electrode (113) includes two longitudinally
extending, laterally spaced-apart legs extending along the length of first jaw (112). A second jaw (122) is positioned at the distal end of second arm (120); while a finger ring (124) is positioned at the proximal end of second arm (120). As shown in FIG. 4, second jaw (122) includes an electrode (123). Electrode (123) is U-shaped in the present example, with the bend of the U-shape located near the distal end of second jaw (122), such that electrode (123) includes two longitudinally extending, laterally spaced-apart legs extending along the length of second jaw (122).

A cable (130) also extends from the proximal end of second arm (120). Cable (130) is coupled with a control unit (132), which is further coupled with a power source (134). Control unit (132) and power source (134) are operable to provide RF power to electrodes (113, 123) in jaws (112, 114), to thereby seal tissue captured between jaws (112, 114). In some versions, control unit (132) comprises a GEN 300 sold by Ethicon Endo-Surgery, Inc. of Cincinnati, Ohio. By way of example only, control unit (132) may be configured in accordance with at least some of the teachings of U.S. Pub. No. 2011/0087212, entitled "Surgical Generator for Ultrasonic and Electrosurgical Devices," published April 14, 2011. A pivoting trigger (136) is positioned in second arm (120) and is operable to selectively switch the RF power to electrodes (113, 123) on and off.

A link (140) is pivotally coupled with first arm (110) by a pin (142). A firing beam (150) is pivotally coupled with link (140) by a pin (144). Firing beam (150) extends into jaws (112, 122) and includes a distal cutting edge (152) that is operable to sever tissue captured between jaws (112, 122) as will be described in greater detail below. Firing beam (150) also includes a lower flange (154) and an upper flange (156), which are configured to bear against opposing surfaces of jaws (112, 122) to maintain jaws (112, 122) in a closed position as firing beam (150) translates distally through jaws (112, 122). As best seen in FIG. 3, lower flange (154) is configured to translate through a channel (114) formed in jaw (112) while upper flange (156) is configured to translate through a channel (124) formed in jaw (122). Channel (124) includes an entry region (126) that is configured to allow jaws (112, 122) to pivot to an open configuration; and to
allow flange (156) to enter channel (124) when jaws (112, 122) are pivoted to a closed configuration.

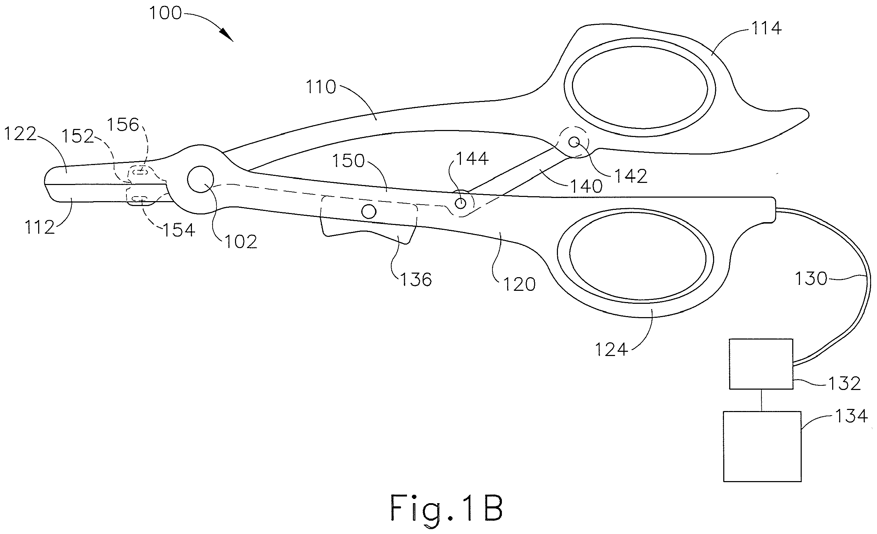

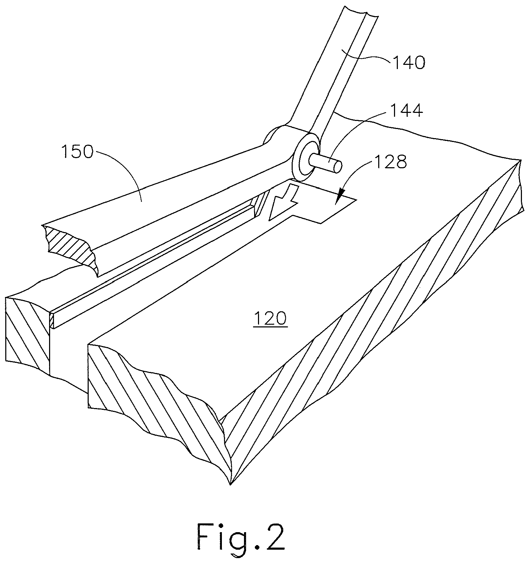

FIGS. 1A-1C show instrument (100) at different stages during operation. In the transition from the configuration shown in FIG. 1A to the configuration shown in FIG. 1B, first arm (110) is pivoted toward second arm (120) to transition jaws from the open position to the closed position. During this motion, pin (144) enters a channel (128) formed in second arm (120), as best seen in FIG. 2. Firing beam (150) also fully seats within channel (128). Instrument (100) may be configured such that trigger (136) is unable to activate electrodes (113, 123) until jaws (112, 122) are closed as shown in FIG. 1B. For instance, this may be accomplished using a mechanical lockout (e.g., preventing movement of trigger (136)) and/or using an electrical lockout (e.g., preventing closure of a circuit when trigger (136) is moved). Various suitable ways in which a trigger (136) lockout may be provided will be apparent to those of ordinary skill in the art in view of the teachings herein.

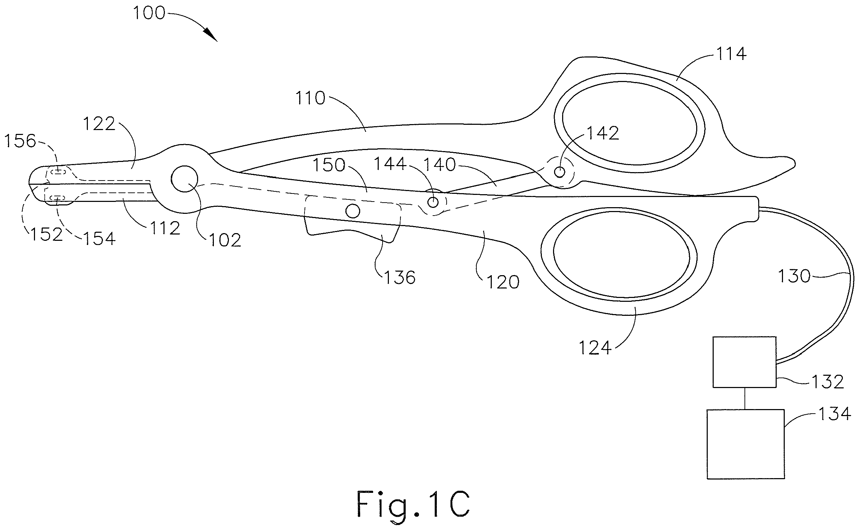

In some instances of use, an operator may simply wish to grasp and perhaps seal tissue with jaws (112, 122). In such instances, the operator may release their grip on rings (114, 124) after reaching the configuration shown in FIG. 1B, and thereby return to the open configuration shown in FIG. 1A. In some other instances, the operator may wish to sever the tissue in jaws (112, 122). To that end, the operator may squeeze rings (114, 124) further toward each other. When sufficient force is applied, first arm (110) deforms to the configuration shown in FIG. 1C. As a result of such deformation, link (140) pivots and drives firing beam (150) distally. Thus, distal cutting edge (152) severs tissue captured between jaws (112, 122). It should be understood that the configuration of instrument (100) as shown may substantially prevent firing beam (150) from advancing distally until jaws (112, 122) have reached a closed configuration. To retract firing beam (150) and open jaws (112, 122) after reaching the stage shown in FIG. 1C, the operator may simply pull rings (114, 124) apart from each other, eventually reaching the configuration shown in FIG. 1A. In some versions, a resilient member (e.g., leaf spring, torsion spring, etc.) may be used to resiliently bias arms (110, 120) and jaws (112, 122) to the open configuration shown in FIG. 1A. In addition or in the alternative, resilience of first arm (110) may at least bias arms (110, 120) and jaws (112, 122) from the configuration shown in FIG. 1C to the configuration shown in FIG. 1B. It should also be understood that electrodes (113, 123) may remain activated with RF energy during at least part of the distal travel of firing beam (150).

### B. Exemplary Electrosurgical Forceps with Rack-Driven Firing Beam

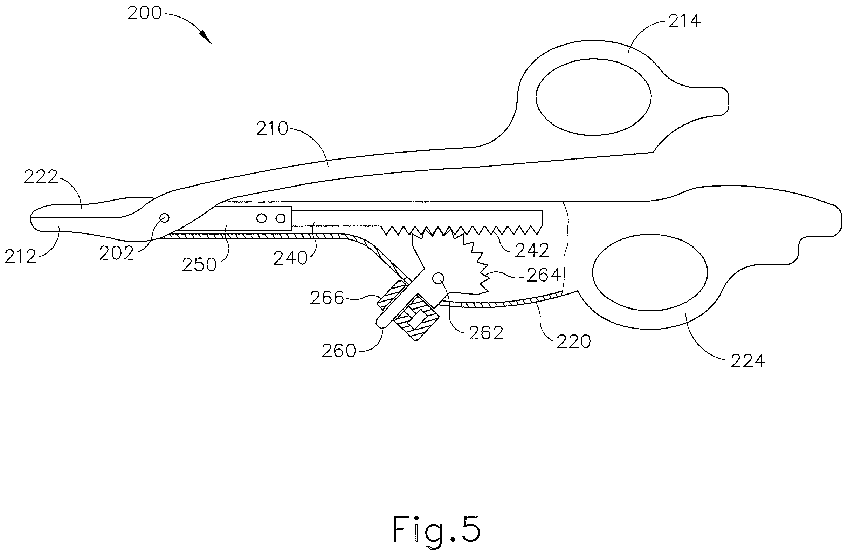

FIG. 5 shows another exemplary electrosurgical forceps instrument (200) that may be used to grasp tissue, seal tissue, and sever tissue. Instrument (200) of this example includes a first arm (210) and a second arm (220) that are pivotally coupled by a pin (202). A first jaw (212) is positioned at the distal end of first arm (210); while a thumb ring (214) is positioned at the proximal end of first arm (210). A second jaw (222) is positioned at the distal end of second arm (220); while a finger ring (224) is positioned at the proximal end of second arm (220). Jaws (212, 222) include electrodes (not shown) that are similar to electrodes (113, 123) described above. Instrument (200) may also include a cable coupled with a control unit and power source, similar to cable (130), control unit (132), and power source (134) described above.

A pivoting trigger (260) is pivotally coupled with second arm (220) by a pin (262). Trigger (260) includes a set of teeth (264) that are positioned along an arcuate path to provide a pinion. Trigger (260) also includes a button assembly (266). Button assembly (266) is operable to selectively activate the electrodes of jaws (212, 222) with RF energy. In the present example, button assembly (266) is configured such that when an operator depresses button assembly (266), the electrodes of jaws (212, 222) will be activated with RF energy before trigger (260) pivots about pin (262).

Teeth (264) of trigger (260) mesh with complementary teeth (242) of a rack (240). Rack (240) is slidably disposed in second arm (220). Rack (240) is secured to a firing beam (250), which is substantially similar to firing beam (150) described above. It should therefore be understood that pivoting of trigger (260) about pin (262) will drive firing beam (250) longitudinally. Thus, if an operator wishes to grasp tissue with instrument (200), the operator may position the tissue between jaws (212, 222) and move ring (214) toward ring (224). If the operator wishes to seal tissue with instrument (200), the operator may depress button assembly (266), which will activate the electrodes of jaws (212, 222) with RF energy. If the operator wishes to sever the tissue with instrument (200), the operator may depress trigger (260), which will drive firing beam (250) distally. In some versions, this may require pressing on button assembly (266) with a force that is greater than the force required to activate the electrodes of jaws (212, 222).

### C. Exemplary Electrosurgical Forceps with Two-Stage Pivoting Trigger

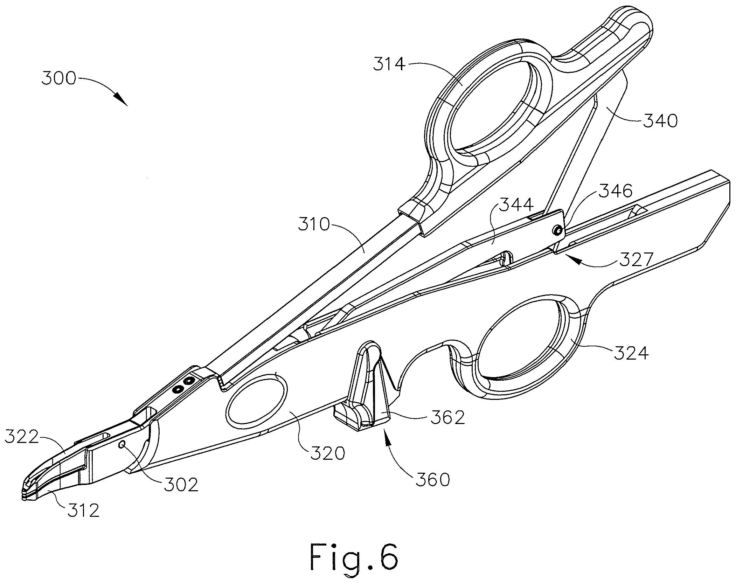

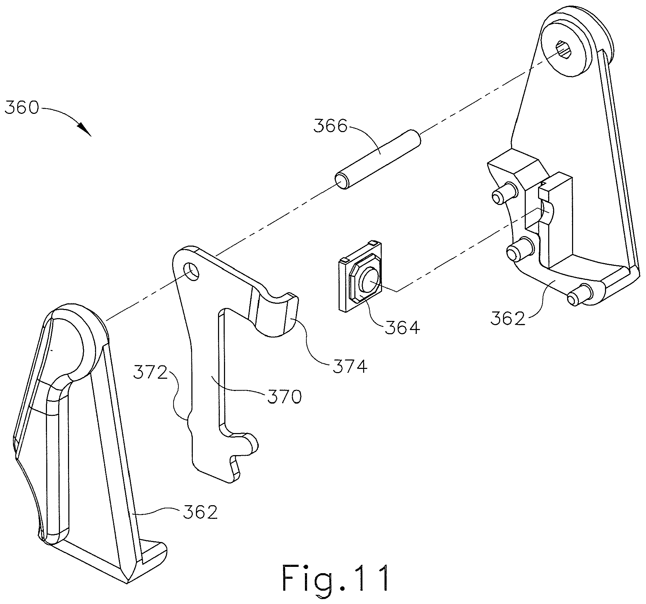

FIGS. 6-13D show yet another exemplary electrosurgical instrument (300) that may be used to grasp tissue, seal tissue, and sever tissue. Instrument (300) of this example includes a first arm (310) and a second arm (320) that are pivotally coupled by a pin (302). A first jaw (312) is positioned at the distal end of first arm (310); while a thumb ring (314) is positioned at the proximal end of first arm (310). A second jaw (322) is positioned at the distal end of second arm (320); while a finger ring (324) is positioned at the proximal end of second arm (320). Jaws (312, 322) include electrodes (not shown) that are similar to electrodes (113, 123) described above. Instrument (300) may also include a cable coupled with a control unit and power source, similar to cable (130), control unit (132), and power source (134) described above.

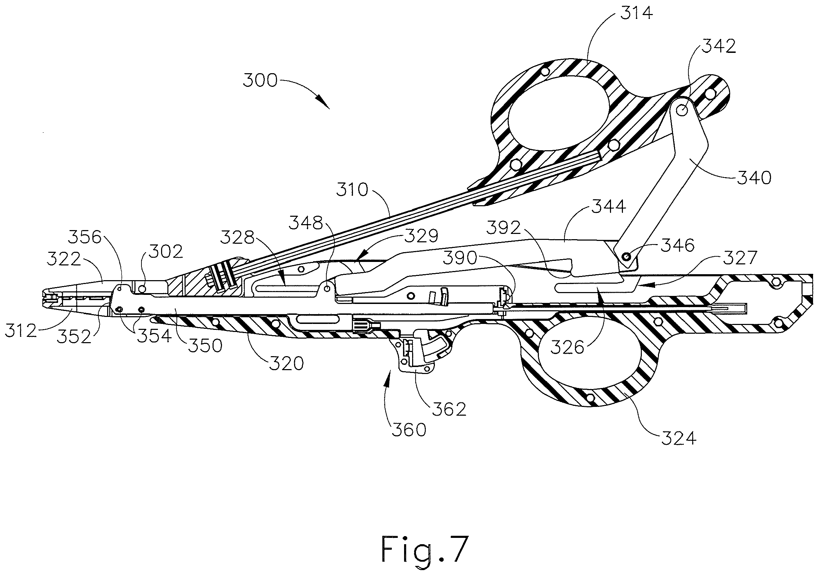

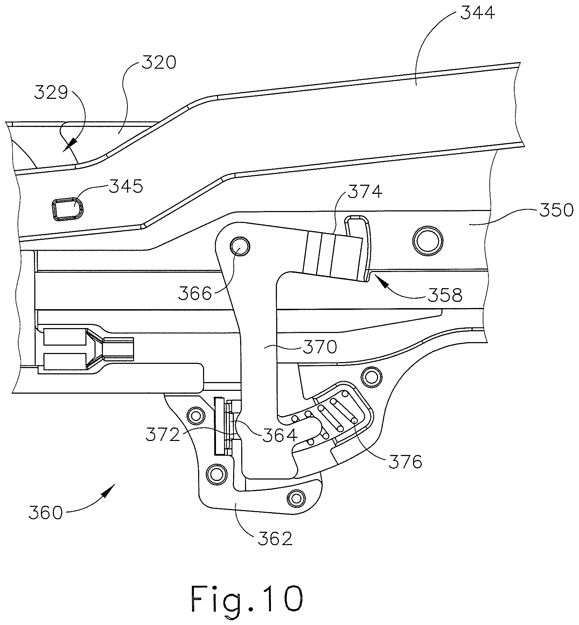

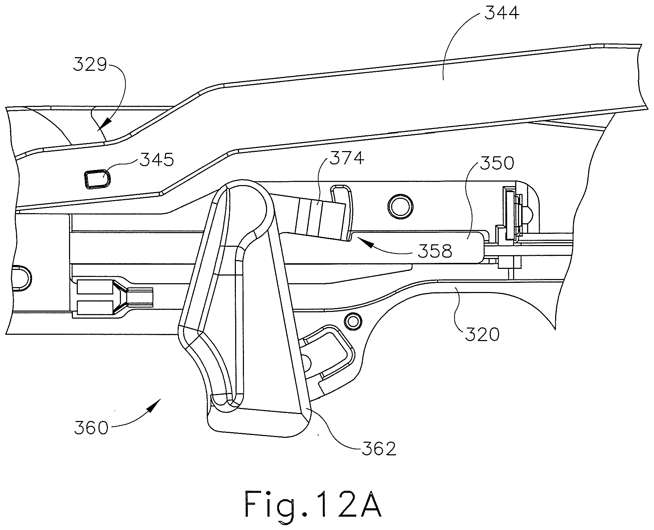

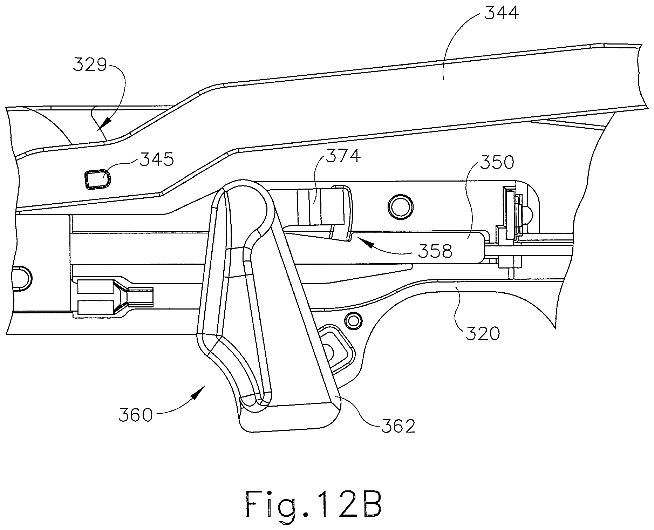

As best seen in FIG. 7, a first link (340) is pivotally coupled with first arm (310) by a pin (342). A second link (344) is pivotally coupled with first link (340) by a pin (346). Second link (344) is also pivotally coupled with a firing beam (350) by a pin (348). Firing beam (350) extends into jaws (312, 322) and includes a distal cutting edge (352) that is operable to sever tissue captured between jaws (312, 322) as will be described in greater detail below. Firing beam (350) also includes a pair of lower transverse pins (354) and an upper transverse pin (356), which are substantially similar in operation to flanges (154, 156) described above. As best seen in FIGS. 10 and 12A-12B, firing beam (350) also includes a notch (358) in a proximal region of firing beam (350). Notch (358) is configured to provide selective locking of firing beam (350) as will be described in greater detail below.

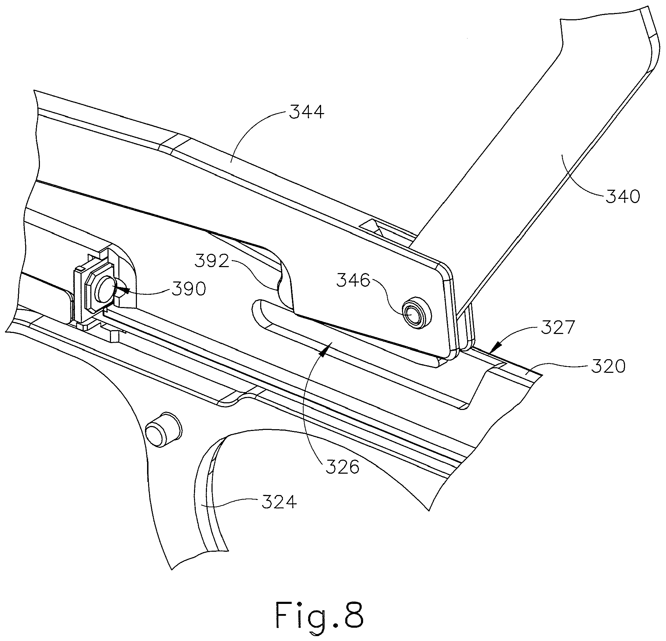

As best seen in FIG. 8, second arm (320) includes a longitudinally extending channel (326) that is configured to slidingly receive pin (346). An entry channel (327) is configured to initially receive pin (346) as arm (310) is pivoted toward arm (320), allowing pin (346) to enter channel (326). As will be described in greater detail below, pin (346) slides distally through channel (326) as firing beam (350) advances distally through jaws (312, 322).

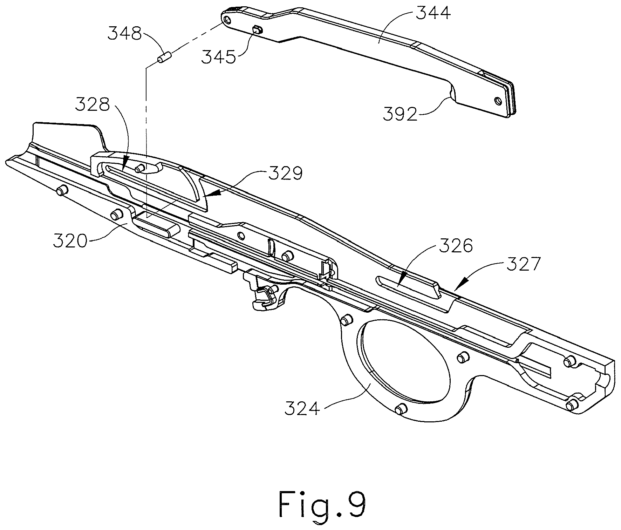

As best seen in FIG. 9, second arm (320) also includes a longitudinally extending channel (328) that is configured to pivotably and slidingly receive pin (348). As will be described in greater detail below, pin (348) slides distally through channel (328) as firing beam (350) advances distally through jaws (312, 322). Channel (328) is also configured to slidingly receive a lateral protrusion (345) of second link (344). Protrusion (345) is initially received in an entry channel (329), even when arms (310, 320) and jaws (312, 322) are in open positions. Entry channel (329) is configured to allow protrusion (345) to eventually enter channel (328) as arm (310) is pivoted further toward arm (320) as will be described in greater detail below. It should be understood that the location of protrusion (345) within entry channel (329) will prevent firing beam (350) from moving distally. In other words, firing beam (350) may only move distally once protrusion (345) reaches the bottom of entry channel (329) where protrusion (345) is free to slide distally in channel (328). Protrusion (345) will slide distally through channel (328) as firing beam (350) advances distally through jaws (312, 322).

It should also be understood that FIGS. 7-8 show just one housing half of second arm (320). The other housing half may have include mirror images of channels (326, 327, 328, 329). Similarly, pin (346) may extend outwardly relative to both sides of link (344); and each side of link (344) may include an identical protrusion (345).

Instrument (300) of the present example also includes a trigger assembly (360) that is operable to selectively activate electrodes in jaws (312, 322) with RF energy and unlock firing beam (350). FIGS. 10-12B show trigger assembly (360) in greater detail. Trigger assembly (360) comprises a housing (362) that is formed by two halves, a dome switch (364), and a pin (366) pivotally coupling housing (362) with second arm (320). Trigger assembly (360) also includes a pivoting lock member (370) positioned within housing (362). Lock member (370) is also pivotally disposed on pin (366). Lock member (370) includes a distal protrusion (372) and a locking arm (374). As shown in FIG. 10, a coil spring (376) is positioned between lock member (370) and second arm (320). Coil spring (376) resiliently biases lock member (370) to rotate clockwise about pin (366) (in the view shown in FIG. 10). As also shown in FIG. 10, locking arm (374) is configured to engage notch (358) of firing beam (350) when lock member (370) is rotated to the clockwise position. This engagement is configured to prevent firing beam (350) from moving distally until locking arm (374) disengages notch (358).

Trigger assembly (360) is configured such that trigger assembly (360) may be actuated in two stages, through two ranges of motion about pin (366). When housing (362) is pulled by the operator through a first range of motion about pin (366), housing (362) drives dome switch (364) into protrusion (372). The spring constant of spring (376) is greater than the spring constant of dome switch (364), such that dome switch (364) is actuated by protrusion (372) before lock member (370) moves. Thus, dome switch (364) is actuated upon completion of the first range of motion of trigger assembly (360). This causes RF energy to be delivered to electrodes in jaws (312, 322). As the operator continues to press housing (362) through a second range of motion about pin (366), housing (362) and dome switch (364) bear against lock member (370) to the point where lock member (370) begins to pivot about pin (366). This eventually causes locking arm (374) to disengage notch (358), as can be seen in the transition from FIG. 12A to the FIG. 12B. With locking arm (374) disengaged from notch (358), firing beam (350) may be translated distally. It should therefore be understood that the electrodes in jaws (312, 322) will be activated with RF energy before firing beam (350) may be advanced distally in this example.

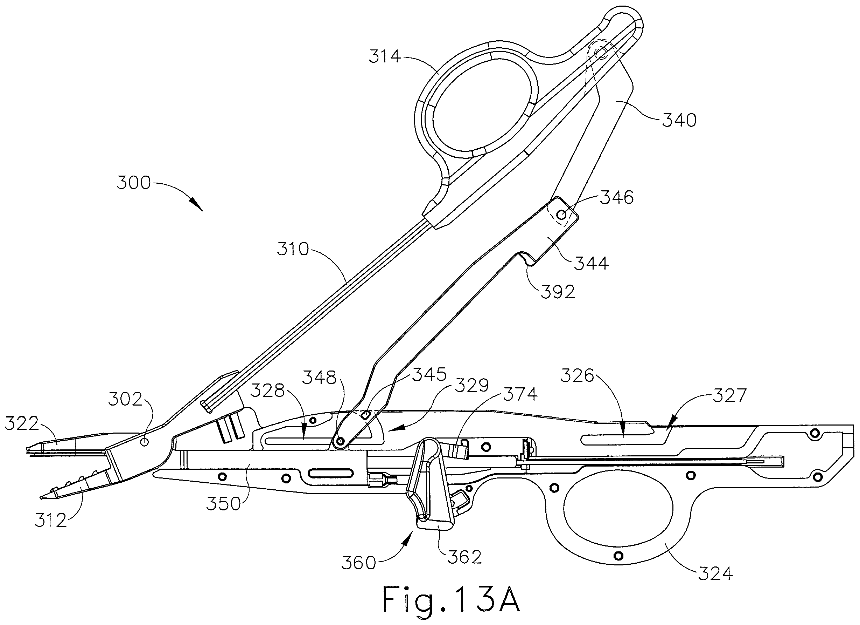

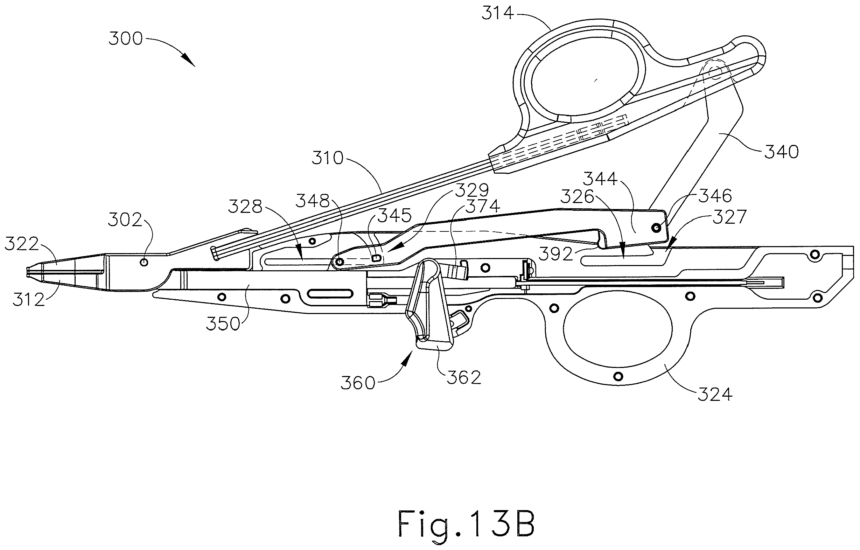

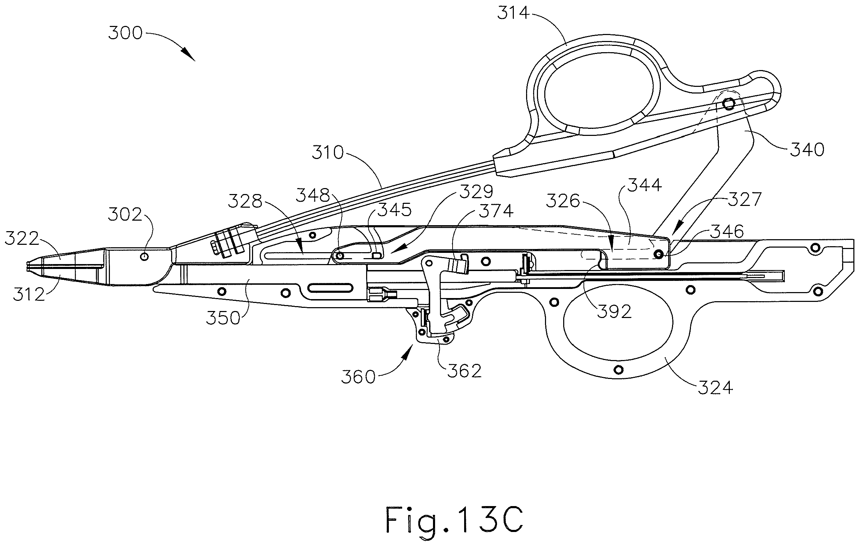

FIGS. 13A-13D show instrument (300) at various stages of operation. In particular, FIG. 13A shows instrument (300) with arms (310, 320) and jaws (312, 322) in fully open positions. With instrument (300) in this configuration, tissue may be positioned between jaws (312, 322). It should be understood that firing beam (350) will not translate at this stage due to engagement of locking arm (374) in notch (358). In addition, the location of protrusion (345) in channel (329) will prevent firing beam (350) from traveling distally at this stage. Once tissue is suitably positioned between jaws (312, 322), rings (314, 324) may be squeezed to pivot arm (310) toward arm (320), thereby pivoting jaw (312) toward jaw (322) to capture tissue between jaws (312, 322). Instrument (300) may thus be configured with closed jaws (312, 322) similar to what is shown in FIG. 13B at this stage (though tissue is not shown in FIG. 13B). With tissue captured between closed jaws (312, 322), the operator may continue squeezing rings (314, 324) to compress the tissue between jaws (312, 322), until arms (310, 320) reach the configuration shown in FIG. 13C. In this configuration, arm (310) is slightly deformed, protrusion (345) has reached the bottom of entry channel (324), and pin (346) has reached the bottom of entry channel (327). This "bottoming out" of protrusion (345) and pin (346) may provide the operator with tactile feedback indicating that the tissue captured between jaws (312, 322) is compressed. The operator may then press trigger assembly (360) through a first range of motion to activate dome switch (364), thereby providing RF energy to electrodes in jaws (312, 322) to seal the tissue. As shown in FIG. 13C, firing beam (350) remains locked at this stage, as trigger assembly (360) has not yet moved lock member (370). This locking of firing beam (350) will also effectively lock links (340, 344) at this stage.

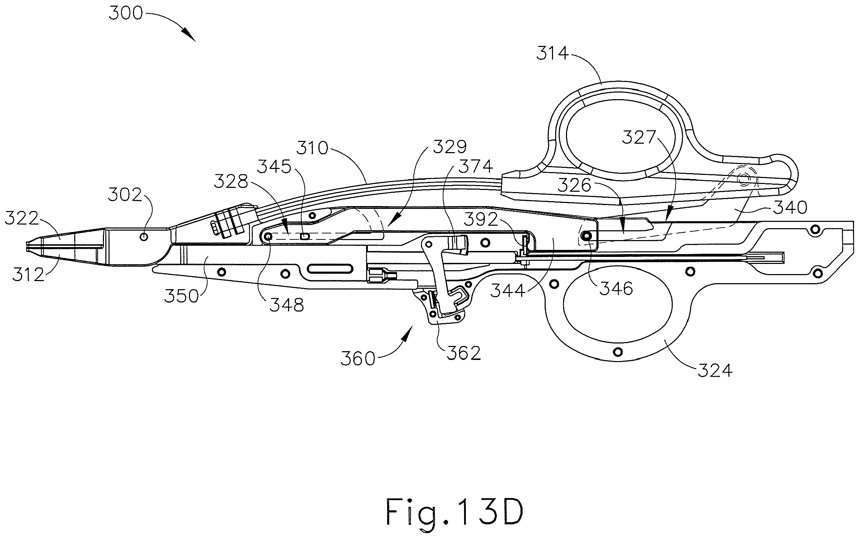

After reaching the stage shown in FIG. 13C, and having at least started the sealing process on tissue captured between jaws (312, 322), the operator may press trigger assembly (360) through the second range of motion to disengage locking arm (374) from notch (358) as shown in FIG. 13D. This unlocks firing beam (350); and further effectively unlocks links (340, 344). The operator may then squeeze rings (314, 324) further, deforming first arm further (310) as also shown in FIG. 13D. This causes links (340, 344) to transition from a generally folded configuration to a generally straight configuration, which drives firing beam (350) distally through jaws (312, 322) to sever the tissue captured between jaws (312, 322). Pin (348) and protrusion (345) travel distally through channel (328) during the transition from the configuration shown in FIG. 13C to the configuration shown in FIG. 13D. Similarly, pin (346) travels distally through channel (326) during the transition from the configuration shown in FIG. 13C to the configuration shown in FIG. 13D. It should be understood that the positioning of pins (346, 348) and protrusion (345) in channels (326, 328) may ensure that links (340, 344) and firing beam (350) remain guided along distally translating paths during the actuation stroke of firing beam (350).

Once firing beam (350) has reached a full range of distal travel as shown in FIG. 13D, a distal protrusion (392) of link (344) engages a proximally facing dome switch (390). Dome switch (390) and protrusion (392) are best seen in FIG. 8. The end-of-stroke engagement between distal protrusion (392) and dome switch (390) turns off the RF energy at electrodes of jaws (312, 322). Of course, this feature is merely optional. For instance, the RF energy may remain active until the operator releases trigger assembly (360) to the point where dome switch (364) disengages protrusion (372). In either case, after completing a firing beam (350) actuation stroke as described above, the operator may release trigger assembly (360), release their grip on rings (314, 324), and pull rings (314, 324) apart until returning to an open position substantially similar to that shown in FIG. 13A. Once firing beam (350) reaches a proximal position again, spring (376) resiliently drives locking arm (374) back into notch (358), thus again locking firing beam (350) in place.

In some exemplary uses, the operator may hold trigger assembly (360) in an actuated position, leaving firing beam (350) unlocked as the operator repeatedly squeezes and releases rings (314, 324). This may enable the operator to repeatedly open and close jaws (312, 322) on tissue. In some instances, the operator may stop short during each squeezing action, such that the operator just seals tissue each time the operator squeezes rings (314, 324). In some other instances, the operator may repeatedly squeeze rings (314, 324) through full actuation strokes, driving firing beam (350) distally each time. In other words, the operator may cut a long continuous line through tissue by repeatedly squeezing and releasing rings (314, 324), using instrument (300) like a conventional set of shears. Other suitable ways in which instrument (300) may be used will be apparent to those of ordinary skill in the art in view of the teachings herein.

### II. Exemplary Electrosurgical Forceps with Disposable Cartridge Feature

Some versions of electrosurgical instruments may include one or more components that are reusable, with other components that are intended to be disposed of after a single use. By way of example only, electronic and/or metallic components of a surgical instrument may be reused due to cost concerns, environmental concerns, and/or other concerns. In view of the foregoing, it may be desirable to enable an operator of a surgical instrument to separate disposable components of the surgical instrument from reusable components of the surgical instrument with relative ease. This would enable the operator to easily dispose of the disposable components and have the reusable components be sterilized and otherwise processed for reuse. In some instances, disposable components may be provided as cartridges that are selectively loaded on reusable components of surgical instruments. Various illustrative examples of such combinations are described in greater detail below; while other examples will be apparent to those of ordinary skill in the art in view of the teachings herein.

### A. Exemplary Electrosurgical Forceps with Cartridge having Sliding Overtube Coupling

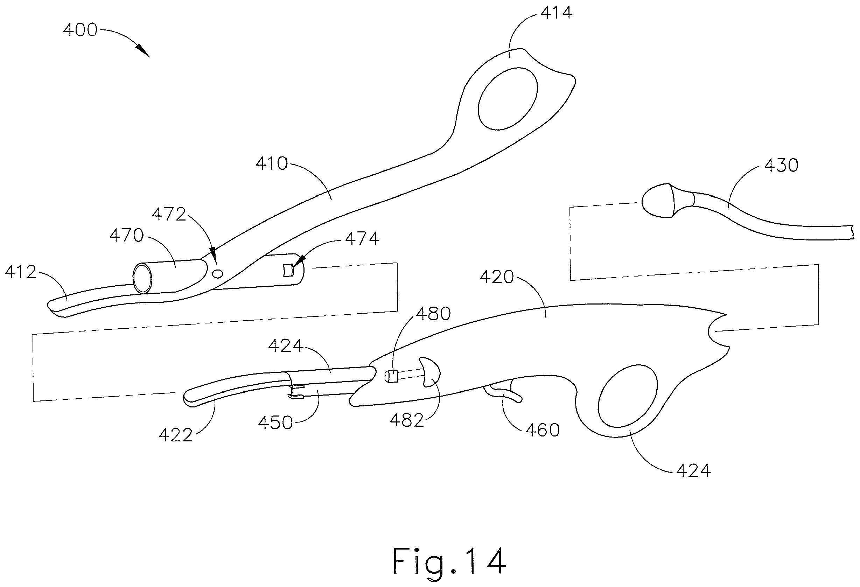

FIG. 14 shows an exemplary electrosurgical instrument (400) that may be used to grasp tissue, seal tissue, and sever tissue. Instrument (400) of this example includes a first arm (410) and a second arm (420) that are configured to selectively couple with each other via a sleeve (470). A first jaw (412) is positioned at the distal end of first arm (410); while a thumb ring (414) is positioned at the proximal end of first arm (410). A second jaw (422) is positioned at the distal end of second arm (420); while a finger ring (424) is positioned at the proximal end of second arm (420). Jaws (412, 422) include electrodes (not shown) that are similar to electrodes (113, 123) described above. Second arm (410) may removably coupled with a cable (430), which may be further coupled with a control unit and power source, similar to control unit (132) and power source (134) described above. A pivoting trigger (460) is also coupled with second arm (410). Trigger (460) is operable to selectively switch the RF power to the electrodes of jaws (412, 422) on and off.

First arm (410) is pivotably coupled with sleeve (470) by a joint (472). The proximal end of sleeve (470) includes a pair of lateral notches (474). Sleeve (470) is configured to slidingly receive jaw (422). Jaw (422) is positioned at the distal end of a support member (424), which is also configured to fit within sleeve (470). A firing beam (450) also fits in sleeve (470). Firing beam (450) is configured and operable similar to firing beam (150) described above, such that firing beam (450) is operable to sever tissue captured between jaws (412, 422). Various suitable ways in which firing beam (450) may be selectively advanced and retracted through jaws (412, 422) will be apparent to those of ordinary skill in the art in view of the teachings herein. When jaw (422), support member (424), and firing beam (450) are inserted through sleeve (470) such that jaw (412) is adjacent to jaw (422), latches (480) snap into lateral notches (474). In the present example, latches (480) are resiliently biased to snap into lateral notches (474). This engagement substantially secures arms (410, 420) together. A pair of buttons (482) on opposing sides of second arm (420) may be depressed to disengage latches (480) from notches (474).

Thus, first arm (410) may be selectively coupled with second arm (420) for use during a medical procedure; and first arm (410) may then be removed from second arm (420). In the present example, first arm (410) is provided as a reusable component while second arm (420) is provided as a disposable component. My way of example only, first arm (410) may be formed entirely of steel, some other metal, and/or some other kind of material that may be processed and reused repeatedly without adversely impacting performance of first arm (410). Cable (430) may also be provided as a reusable component. Other suitable components, features, variations, and operabilities for instrument (400) will be apparent to those of ordinary skill in the art in view of the teachings herein.

### B. Exemplary Electrosurgical Forceps with Cartridge having Snap Arm

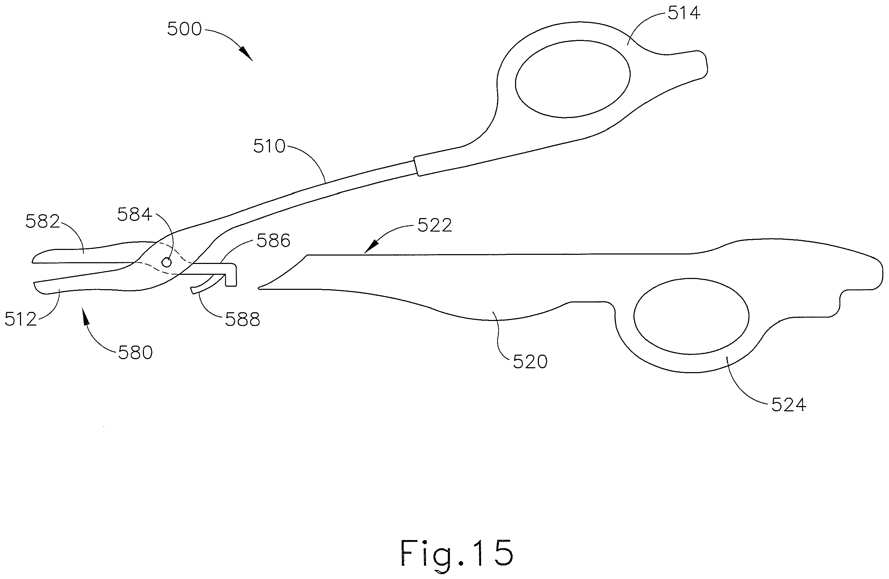

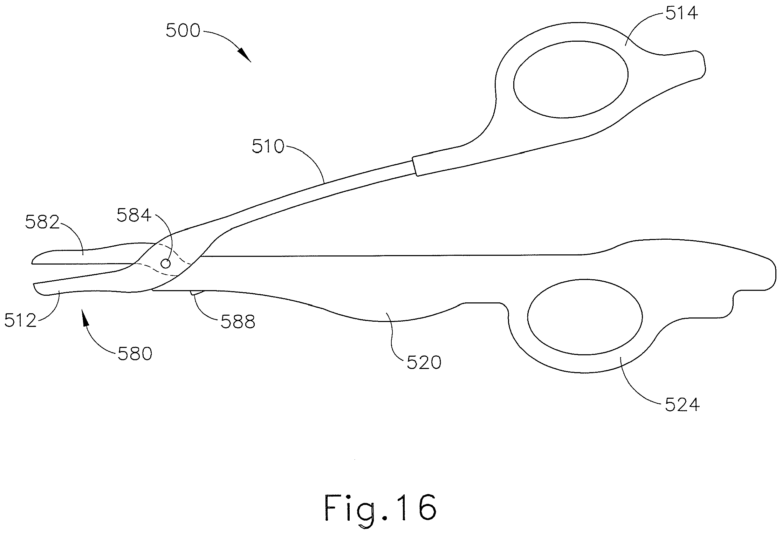

FIGS. 15-18 show another exemplary electrosurgical instrument (500) that may be used to grasp tissue, seal tissue, and sever tissue. Instrument (500) of this example includes a first arm (510) and a second arm (520) that are configured to selectively couple with each other. A jaw assembly (580) is positioned at the distal end of first arm (510); while a thumb ring (514) is positioned at the proximal end of first arm (510). Jaw assembly (580) includes a first jaw (512) and a second jaw (582) that are pivotally coupled by a pin (584). Jaws (512, 582) include electrodes (not shown) that are similar to electrodes (113, 123) described above. A coupling arm (586) extends proximally from second jaw (582). Coupling arm (586) comprises a distally projecting resilient latch (588). Coupling arm (586) is configured to fit in the open distal end (522) of second arm (520). The proximal end of second arm (520) includes a finger ring (524). Instrument (500) may also include a cable coupled with a control unit and power source, similar to cable (130), control unit (132), and power source (134) described above.

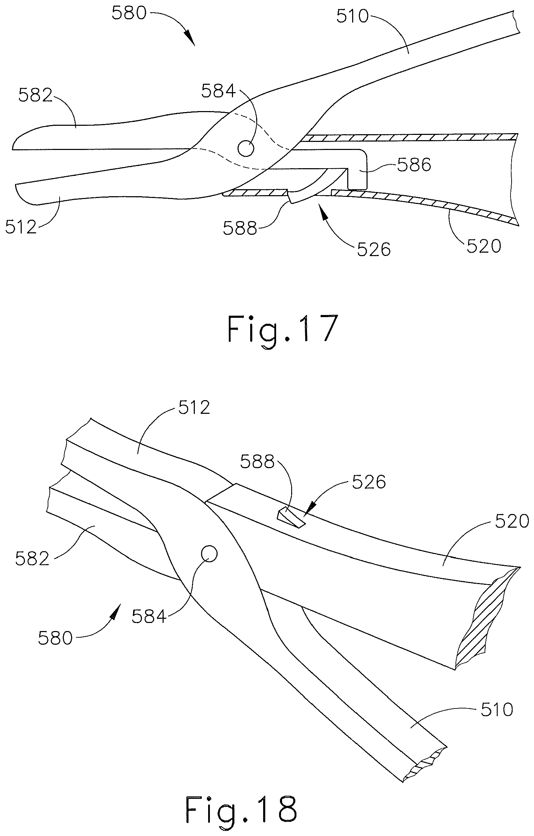

As best seen in FIGS. 17-18, second arm (520) includes a lateral opening (526) that is configured to receive resilient latch (588). As coupling arm (586) is inserted into the open distal end of second arm (520), resilient latch (588) deflects inwardly until latch (588) reaches opening (526). Once latch (588) reaches opening (526), latch (588) resiliently moves outwardly such that a portion of latch (588) protrudes through opening (526). This couples first and second arms (510, 520) together with sufficient strength to use instrument (500) in a medical procedure. Once instrument (500) has been used, first and second arms (510, 520) may be de-coupled by a user pressing the exposed portion of latch (588) inwardly and then pulling arms (510, 520) apart. In the present example, first arm (510) is provided as a disposable component while second arm (520) is provided as a reusable component. Of course, any other suitable relationships may be used. It should also be understood that instrument (500) may include a firing beam similar to any of the firing beams described herein, a trigger similar to any of the triggers described herein, and/or numerous other components and features. Other suitable components, features, variations, and operabilities for instrument (500) will be apparent to those of ordinary skill in the art in view of the teachings herein.

### C. Exemplary Electrosurgical Forceps with Cartridge having Vertically Deflecting Resilient Firing Beam Lock

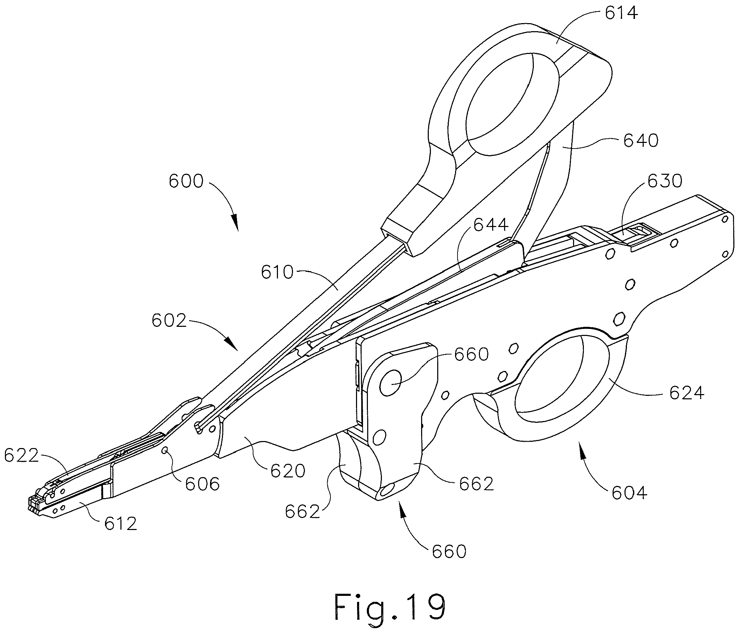

FIGS. 19-26 show another exemplary electrosurgical instrument (600) that may be used to grasp tissue, seal tissue, and sever tissue. Instrument of this example comprises a disposable cartridge portion (602) and a reusable grip portion (604). Cartridge portion (602) and grip portion (604) are releasably coupled together by a resiliently biased latch (630). Cartridge portion (602) includes a first arm (610) and a second arm (620) that are configured to selectively couple with each other. A first jaw (612) is positioned at the distal end of first arm (610); while a thumb ring (614) is positioned at the proximal end of first arm (610). A second jaw (622) is positioned at the distal end of second arm (620). Jaws (612, 622) are pivotally coupled at a pin (606). Jaws (612, 622) include electrodes (not shown) that are similar to electrodes (113, 123) described above. These electrodes receive power through an electrical coupling between cartridge portion (602) and grip portion (604), as will be described in greater detail below. Grip portion (604) may include a cable coupled with a control unit and power source, similar to cable (130), control unit (132), and power source (134) described above.

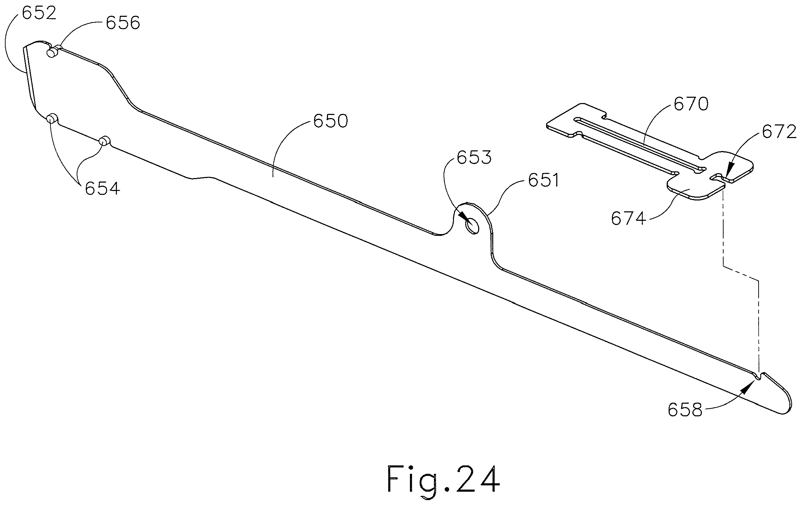

As best seen in FIGS. 19-21, cartridge portion (602) includes a first link (640) that is pivotally coupled with first arm (610) by a pin (not shown). A second link (644) is pivotally coupled with first link (640) by a pin (not shown). Second link (644) is also pivotally coupled with a firing beam (650) by a pin (648). An upper protrusion (651) of firing beam (650) defines an opening (653) that is configured to receive pin (648). Firing beam (650) extends into jaws (612, 622) and includes a distal cutting edge (652) that is operable to sever tissue captured between jaws (612, 622) as will be described in greater detail below. As best seen in FIG. 24, firing beam (650) also includes a pair of lower transverse pins (654) and an upper transverse pin (656), which are substantially similar in operation to flanges (154, 156) described above. Firing beam (650) also includes a catch (658) in a proximal region of firing beam (650). Catch (658) is configured to provide selective locking of firing beam (650). In particular, catch (658) is configured to engage a firing beam locking member (670).

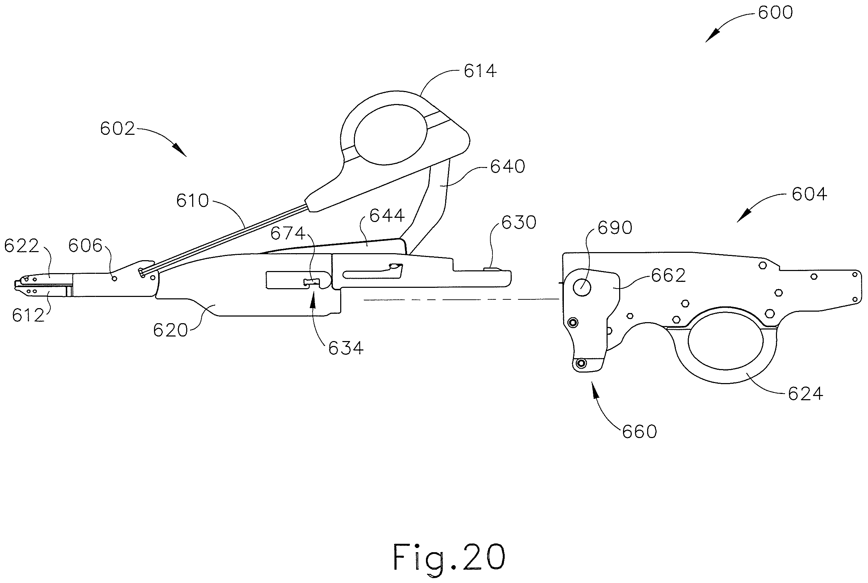

As best seen in FIG. 24, firing beam locking member (670) includes a proximal notch (672) that is configured to receive catch (658). Firing beam locking member (670) also includes outwardly extending tabs (674). Firing beam locking member (670) is positioned over and adjacent to the proximal end of firing beam (650) and is resiliently biased to assume a straight configuration, where firing beam locking member (670) will engage catch (658) to prevent distal translation of firing beam (650). As shown in FIGS. 20-26 and 23, second arm (620) defines openings (634) that are configured to expose tabs (674). This enables tabs (674) to be engaged by other components of instrument (600) as will be described in greater detail below.

In the present example, second arm (620) includes channels (not shown) that are similar to channels (326, 327, 328, 329) described above. For instance, channels in second arm (620) that are similar to channels (326, 327) may receive a pin that couples links (640, 644), similar to pin (346). Likewise, channels in second arm (620) that are similar to channels (328, 329) may receive pin (648) and a lateral protrusion (645) of link (644). When firing beam (650) is unlocked and ring (614) is squeezed toward arm (620), the pins and protrusion (645) slide along the channels to guide links (640, 644) as links (640, 644) approach a substantially straight configuration, thereby advancing firing beam (650) distally through jaws (612, 622).

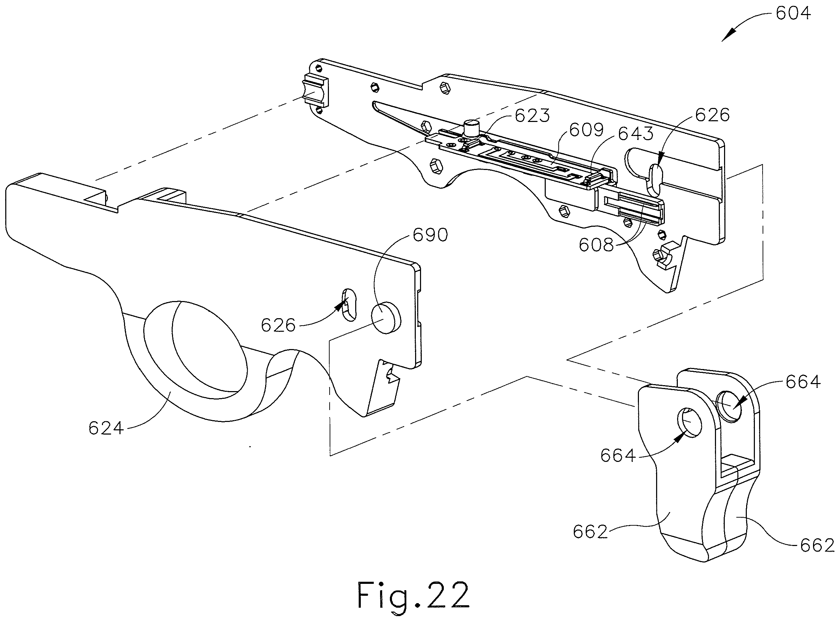

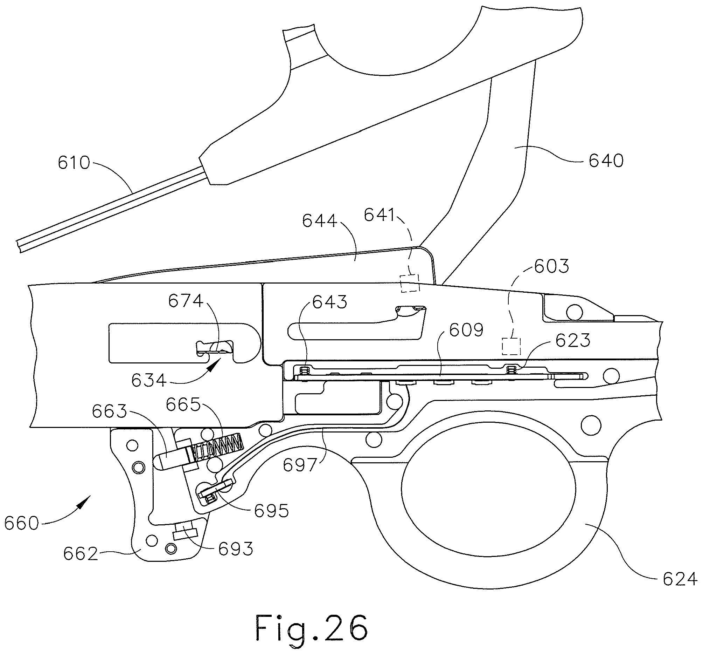

Grip portion (604) of instrument (600) includes a finger ring (624) and a trigger assembly (660) that is operable to activate RF energy at electrodes in jaws (612, 622) and unlock firing beam (650) for distal advancement. Trigger assembly (660) comprises a pair of trigger body halves (662), each half (662) defining a respective opening (664). As best seen in FIG. 22, integral outwardly extending posts (690) of grip portion (604) are disposed in openings (664), providing a pivotal coupling between trigger assembly (660) and grip portion (604). As best seen in FIG. 26, a plunger (663) and spring (665) resiliently bias trigger assembly (660) to an extended position.

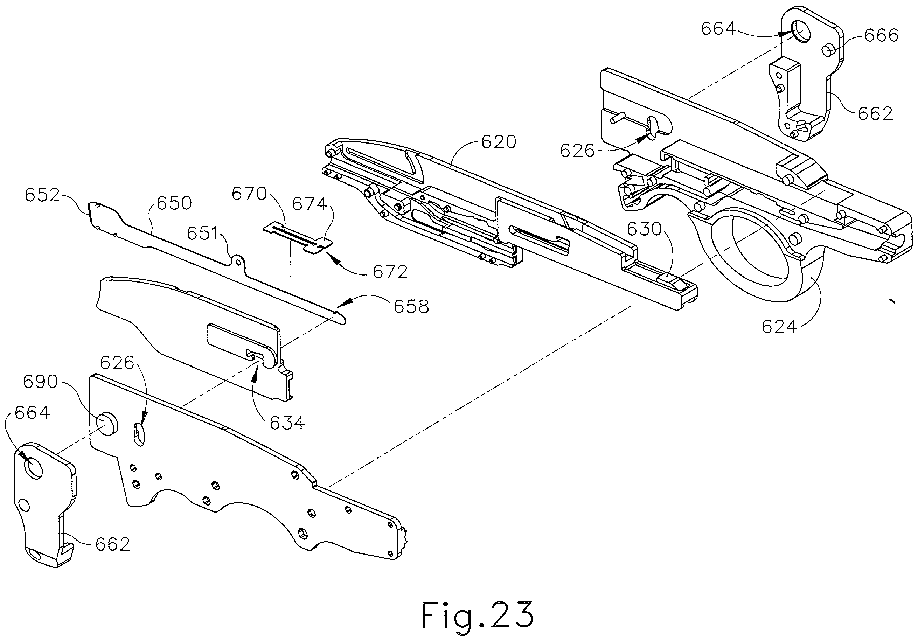

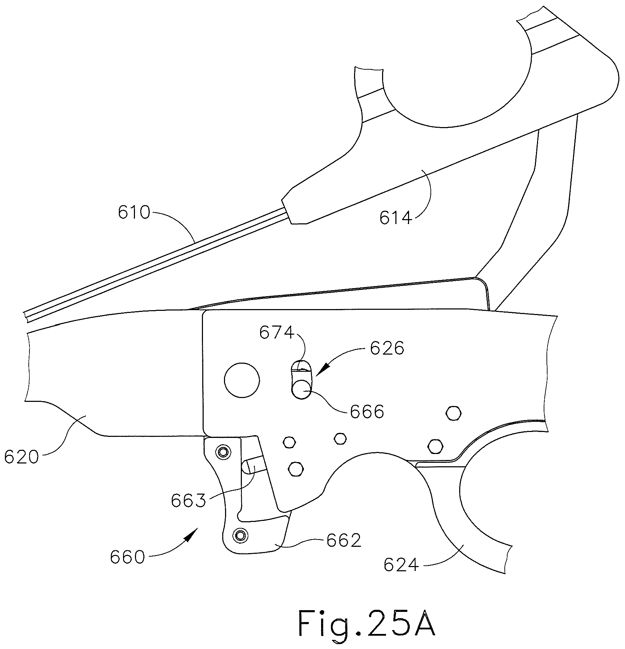

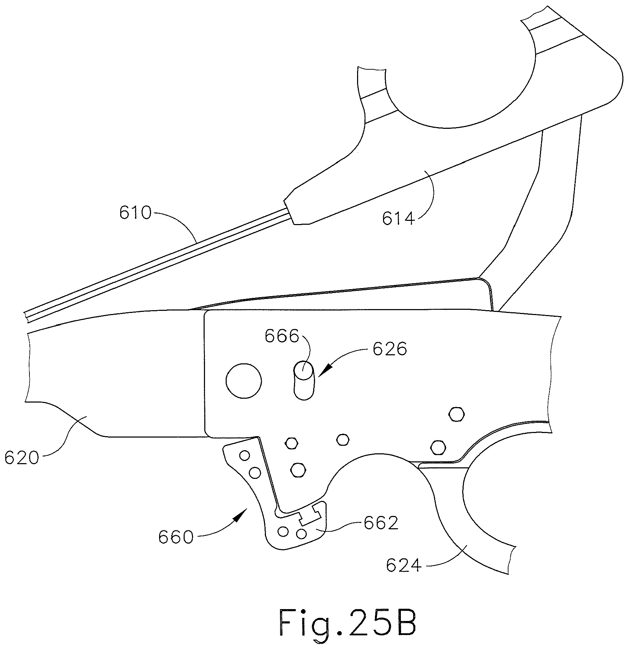

As best seen in FIGS. 23 and 25A-25B, each trigger body half (662) also includes an inwardly extending protrusion (666). Protrusions (666) are slidably received in slots (626) of grip portion (604). Slots (626) are positioned to generally align with openings (634) when cartridge portion (602) is fully seated in grip portion (604). Protrusions (666) are configured to move within slots (626) to selectively engage tabs (674) of firing beam locking member (670). As shown in FIG. 25A, protrusions (666) do not contact tabs (674) when trigger assembly (660) is in the extended position. Firing beam locking member (670) is thus in a substantially straight configuration, such that firing beam locking member (670) prevents distal translation of firing beam (650). When trigger assembly (660) is actuated as shown in FIG. 25B, protrusions (666) slide through slots (626) and engage tabs (674). This deforms firing beam locking member (670) by bending firing beam locking member (670) upwardly, thereby driving tabs (674) upwardly to a point where catch (658) is able to clear firing beam locking member (670).

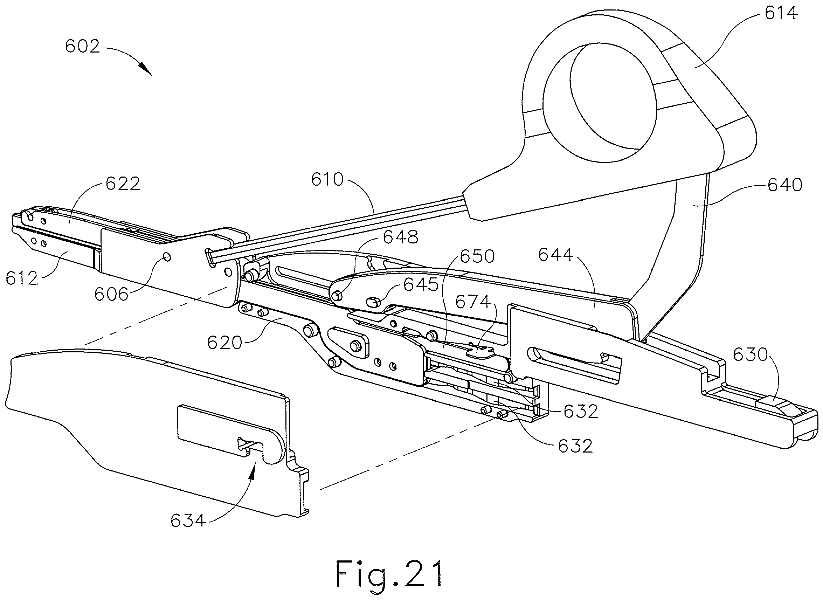

FIGS. 21-22 show features that provide electrical coupling between cartridge portion (602) and grip portion (604) when cartridge portion (602) and grip portion (604) are mechanically coupled together. In particular, FIG. 21 shows a set of contacts (632) that are in electrical communication with electrodes in jaws (612, 622). FIG. 22 shows a set of contacts (608) that are in electrical communication with a circuit board (609) in grip portion (604). Contacts (632) are configured to engage contacts (608) when cartridge portion (602) and grip portion (604) are mechanically coupled together. Contacts (608, 632) thus provide a path for electrical communication between cartridge portion (602) and grip portion (604). Of course, any other suitable features may be used to provide electrical communication between cartridge portion (602) and grip portion (604).

Instrument (600) of the present example also includes a set of contactless electrical features that are configured to drive at least part of the operation of instrument (600). In particular, as best seen in FIG. 26, a pair of reed switches (623, 643) are mounted to circuit board (609) while another reed switch (695) is coupled with circuit board (609) by a conduit (697) (e.g., wire, flex circuit, etc.).

Reed switch (623) is configured to be activated by a magnet (603) that is mounted at the proximal end of cartridge portion (602). In particular, reed switch (623) may be used to detect whether cartridge portion (602) is fully seated in grip portion (604). A control logic in the circuit may be configured to prevent an electrical signal from being sent to contacts (608) in the absence of cartridge portion (602).

Reed switch (643) is configured to be activated by a magnet (641) located near the joint of links (640, 644). Reed switch (643) may be positioned such that magnet (641) activates reed switch (643) as soon as firing beam (650) has been driven to a distal position by links (640, 644). A control logic in the circuit may be configured to cut off RF power to the electrodes in jaws (612, 622) after firing beam (650) reaches the distal position (or after a predetermined time period has elapsed after firing beam (650) reaches the distal position, etc.).

Reed switch (695) is configured to be activated by a magnet (693) in trigger assembly (660). In particular, reed switch (695) may be positioned such that magnet (693) activates reed switch (695) as soon as trigger assembly (660) is fully actuated. A control logic in the circuit may be configured to activate the electrodes in jaws (612, 622) with RF energy once trigger assembly (660) is fully actuated. It should be understood that the configuration of trigger assembly (660) and firing beam locking member (670) will prevent firing beam (650) from advancing distally until after the electrodes in jaws (612, 622) have been activated with RF energy.

In an exemplary use, cartridge portion (602) and grip portion (604) are initially provided as separate components. Second arm (620) of cartridge portion (602) is then inserted into grip portion (604) until latch (630) snaps into place to secure portions (602, 604) together. At this stage, contacts (608, 632) engage each other to provide a path for electrical continuity between portions (602, 604); and magnet (603) cooperates with reed switch (623) to register the coupling of portions (602, 604). Jaws (612, 622) are then positioned at a surgical site in a patient, with tissue between jaws (612, 622). The operator then squeezes rings (614, 624) toward each other to compress the tissue between jaws (612, 622). Once links (644, 640) reach a point where catch (658) bears against firing beam locking member (670), arm (610) can pivot no further toward arm (620). The operator then pivots trigger assembly (660) about posts (690). Magnet (693) eventually trips reed switch (695), which then causes RF energy to be delivered to electrodes in jaws (612, 622). In addition, protrusions (666) drive into tabs (674), deflecting firing beam locking member (670) out of engagement with catch (658). The operator then squeezes rings (614, 640) further, causing links (640, 644) to pivot to generally straight positions, thereby driving firing beam (650) distally. It should be understood that first arm (610) may bend to some degree during this stage. The distally advancing firing beam (650) severs the tissue between jaws (612, 622). Once firing beam (650) reaches the distal position, magnet (641) trips reed switch (643), effectively cutting off the RF energy at the electrodes in jaws (612, 622). The operator then relaxes their grip on rings (614, 640), releasing the tissue from jaws (612, 622) and retracting firing beam (650) proximally.

The above process may be repeated as many times as desired. For instance, jaws (612, 622) and firing beam (650) may be actuated repeatedly along a continuous line for any suitable length. Alternatively, jaws (612, 622) and firing beam (650) may be actuated repeatedly at different tissue sites. It should also be understood that jaws (612, 622) may be used to only grasp tissue, or to only grasp and seal tissue, without necessarily also severing the tissue with firing beam (650). After the operator is done using instrument (600), the operator may depress latch (630) and separate cartridge portion (602) from grip portion (604). The operator may then dispose of cartridge portion (602) and send grip portion (604) through a sterilization/reclamation process. Grip portion (604) may thus be later used in another surgical procedure with another cartridge portion (602). Other suitable components, features, variations, and operabilities for instrument (600) will be apparent to those of ordinary skill in the art in view of the teachings herein.

### D. Exemplary Electrosurgical Forceps with Cartridge having Laterally Deflecting Resilient Firing Beam Lock

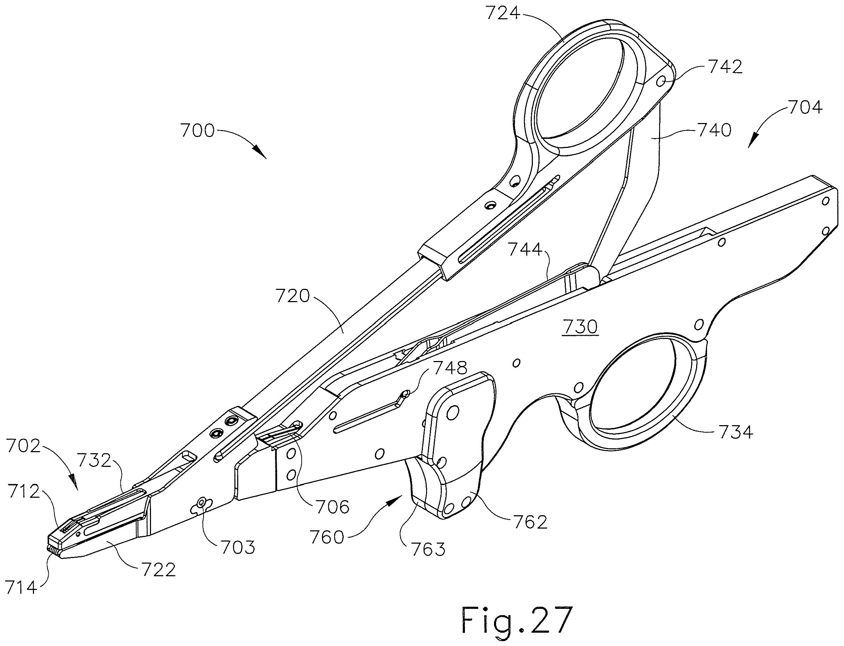

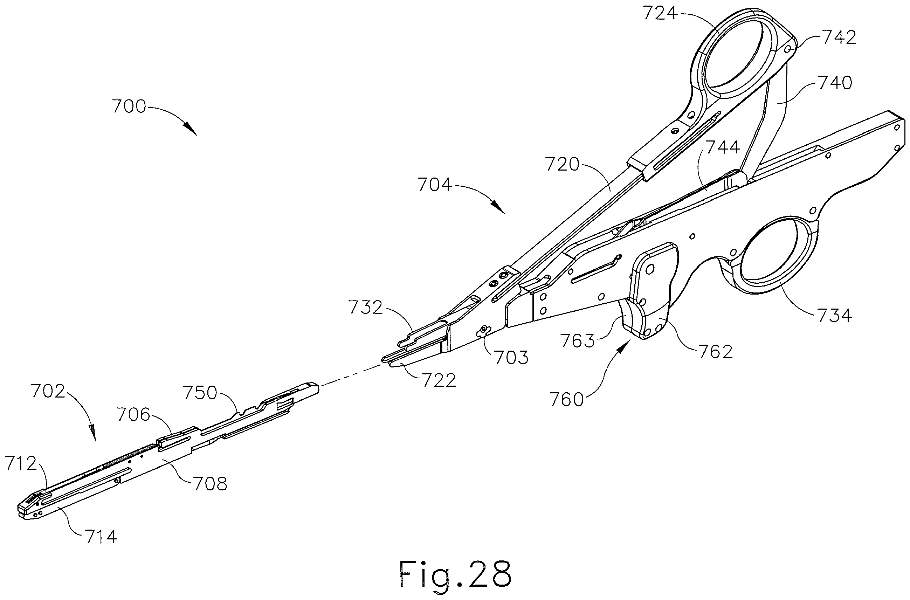

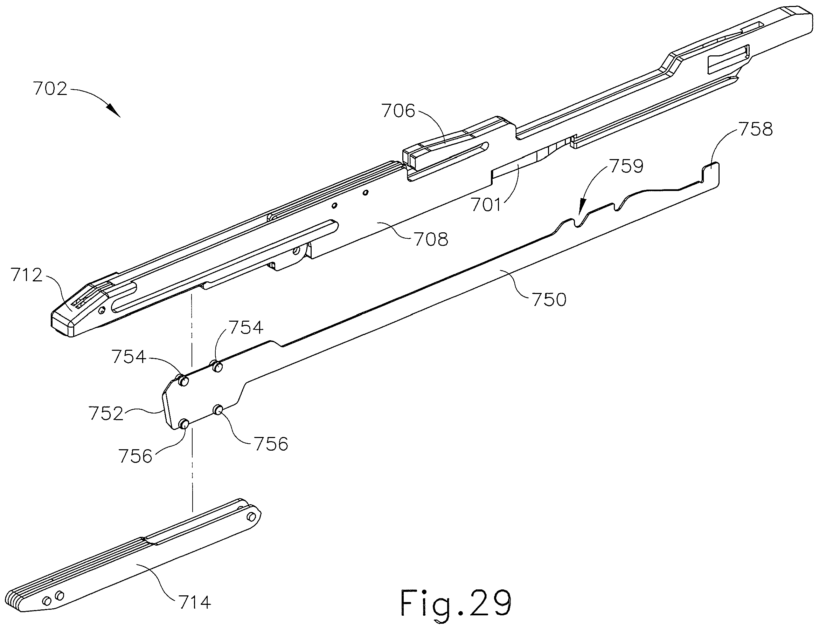

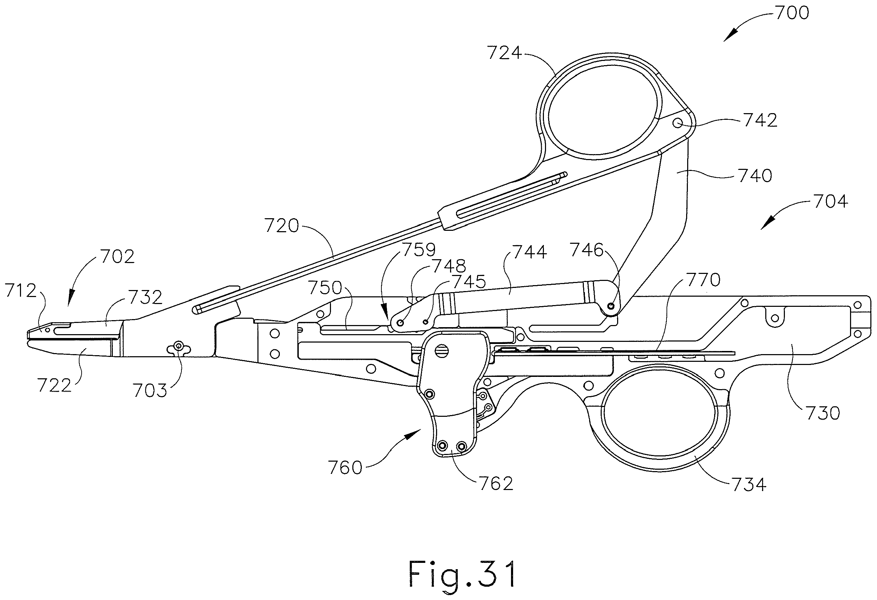

FIGS. 27-34 show another exemplary electrosurgical instrument (700) that may be used to grasp tissue, seal tissue, and sever tissue. Instrument of this example comprises a disposable cartridge portion (702) and a reusable grip portion (704). Cartridge portion (702) and grip portion (704) are releasably coupled together by a resiliently biased latch (706), which is an integral feature of the body (708) of cartridge portion (702). As best seen in FIG. 28, cartridge portion (702) includes a first jaw (712) and a second jaw (714). First jaw (712) is a unitary feature of body (708); while second jaw (714) is pivotably coupled with body (708). Jaws (712, 714) include electrodes (not shown) that are similar to electrodes (113, 123) described above. These electrodes receive power through an electrical coupling between cartridge portion (702) and grip portion (704). In particular, cartridge portion (702) includes exposed contacts (701) (see FIG. 29) that engage complementary contacts (not shown) of grip portion (704) when cartridge portion (702) is fully seated in grip portion (704). Grip portion (704) may include a cable coupled with a control unit and power source, similar to cable (130), control unit (132), and power source (134) described above.

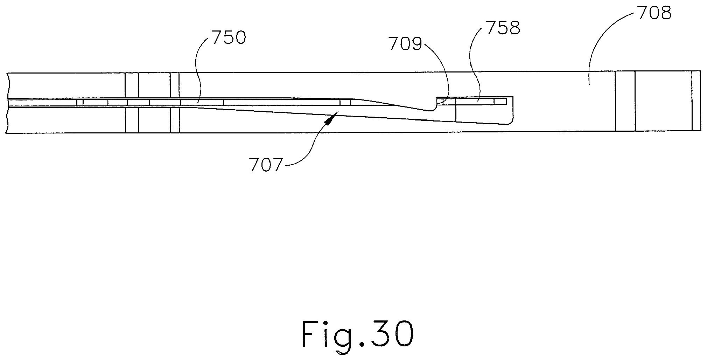

A firing beam (750) is slidably disposed in body (708). Firing beam (750) extends into jaws (712, 714) and includes a distal cutting edge (752) that is operable to sever tissue captured between jaws (712, 714) as will be described in greater detail below. As best seen in FIG. 29, firing beam (750) also includes a pair of upper transverse pins (754) and a pair of lower transverse pins (756), which are substantially similar in operation to flanges (154, 156) described above. Firing beam (750) also includes a catch (758) at the proximal end of firing beam (750). Catch (758) is configured to provide selective locking of firing beam (750). In particular, catch (758) is configured to engage a lateral projection (709) of body (708), as best seen in FIG. 30. Firing beam (750) is resiliently biased to assume the straight configuration shown in FIGS. 29-30, though firing beam (750) is flexible enough to permit catch (758) to be deflected laterally to disengage projection (709) and thereafter translate distally through channel (707) formed in body (708). An example of structure that may be used to deflect catch (758) laterally will be described in greater detail below. Firing beam (750) also includes an upper notch (759) that is used to drive firing beam distally (750), as will also be described in greater detail below.

Grip portion (702) includes a first arm (720) and a second arm (730) that are pivotally coupled by a pin (703). A first jaw support (732) is positioned at the distal end of second arm (730) and is configured to receive and support first jaw (712). A second jaw support (722) is positioned at the distal end of first arm (720) and is configured to receive and support second jaw (714). Thus, second jaw support (722) and second jaw (714) pivot together relative to the combination of first jaw support (732) and first jaw (712). A thumb ring (724) at the proximal end of first arm (720) may be squeezed toward second arm (730) to pivot second jaw support (722) and second jaw (714) toward the combination of first jaw support (732) and first jaw (712). A finger ring (734) of second arm (730) may be held for support during such squeezing of thumb ring (724).

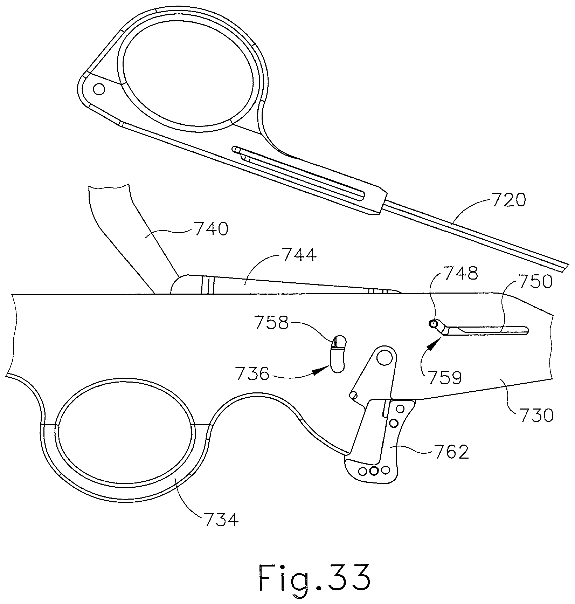

As best seen in FIGS. 27-28, 31, and 34, grip portion (704) includes a first link (740) that is pivotally coupled with first arm (720) by a pin (742). A second link (744) is pivotally coupled with first link (740) by a pin (746). Second link (744) includes a pin (748) that is configured to selectively engage firing beam (750). In particular, upper notch (759) of firing beam (750) is configured to receive pin (748). When first arm (720) is pivoted sufficiently away from second arm (730), pin (748) moves away from notch (759) and provides sufficient clearance for cartridge portion (704) to be coupled with and removed from grip portion (704) (see FIG. 33). Second link (744) also includes a lateral protrusion (745), similar to protrusion (345). Second arm (720) includes channels that are similar to channels (326, 327, 328, 329) described above. For instance, channels in second arm (720) that are similar to channels (326, 327) slidingly receive pin (746). Likewise, channels in second arm (720) that are similar to channels (328, 329) slidingly receive pin (748) and lateral protrusion (745) of link (744). When firing beam (750) is unlocked and ring (724) is squeezed toward arm (720), pins (746, 748) and protrusion (745) slide along the channels to guide links (740, 744) as links (740, 744) approach a substantially straight configuration, thereby advancing firing beam (750) distally through jaws (712, 714).

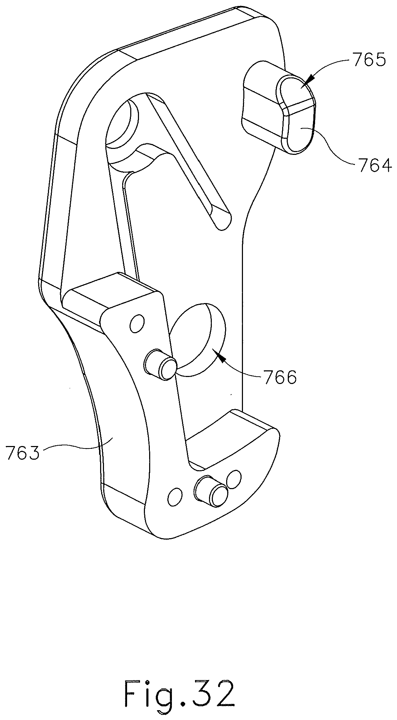

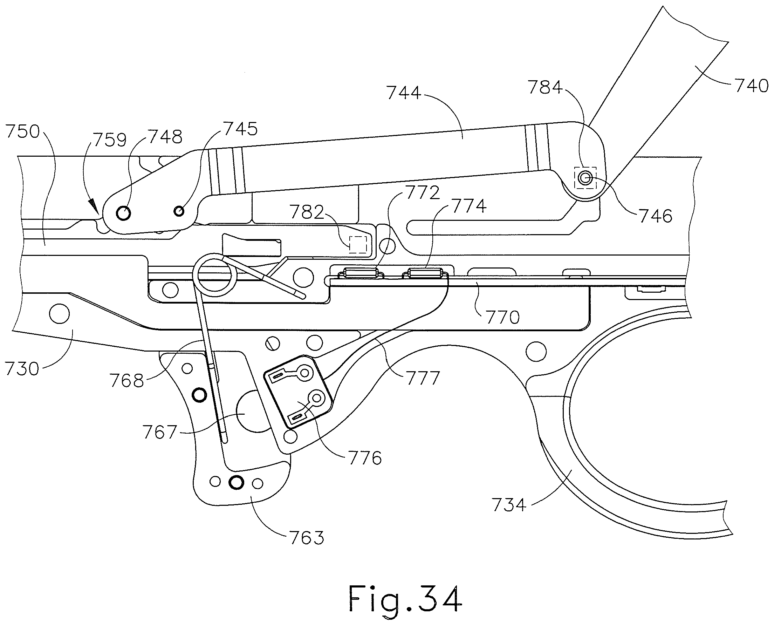

Grip portion (704) also includes a trigger assembly (760) that is operable to activate RF energy at electrodes in jaws (712, 714) and unlock firing beam (750) for distal advancement. Trigger assembly (760) comprises a pair of trigger body halves (762, 763). As best seen in FIG. 32, trigger body half (763) includes a lateral projection (764). Lateral projection (764) is configured to slidingly fit in a slot (736) formed in second arm (730). Slot (736) is positioned such that the upper portion of slot corresponds to the lower portion of catch (758), as shown in FIG. 33. Lateral projection (764) is configured to engage catch (758) when trigger assembly (760) is pivoted relative to second arm (730). Lateral projection (764) includes a chamfer (765) configured to provide a camming action against catch (758). In particular, as trigger assembly (760) is pivoted relative to second arm (730), chamfer (765) cammingly drives catch (758) laterally out of engagement with projection (709). As trigger assembly (760) is held in the pivoted position, projection (764) holds catch (758) in the deflected position, allowing catch (758) to translate distally through channel (707), thereby allowing firing beam (750) to translate through jaws (712, 714). As best seen in FIG. 34, a torsion spring (768) resiliently biases trigger assembly (760) to an extended position.

Instrument (700) of the present example also includes a set of contactless electrical features that are configured to drive at least part of the operation of instrument (700). In particular, as best seen in FIG. 34, a pair of reed switches (772, 774) are mounted to circuit board (770) while another reed switch (776) is coupled with circuit board (770) by a conduit (777) (e.g., wire, flex circuit, etc.).

Reed switch (772) is configured to be activated by a magnet (782) that is mounted at the proximal end of cartridge portion (702). In particular, reed switch (772) may be used to detect whether cartridge portion (702) is fully seated in grip portion (704). A control logic in the circuit may be configured to prevent an electrical signal from being sent to contacts (not shown) that engage contacts (701), in the absence of cartridge portion (702).

Reed switch (774) is configured to be activated by a magnet (784) located near the joint of links (740, 744). Reed switch (774) may be positioned such that magnet (784) activates reed switch (774) as soon as firing beam (750) has been driven to a distal position by links (740, 744). A control logic in the circuit may be configured to cut off RF power to the electrodes in jaws (712, 714) after firing beam (750) reaches the distal position (or after a predetermined time period has elapsed after firing beam (750) reaches the distal position, etc.).

Reed switch (776) is configured to be activated by a magnet (767) that is positioned in a recess (766) formed in trigger body half (763). In particular, reed switch (776) may be positioned such that magnet (767) activates reed switch (776) as soon as trigger assembly (760) is fully actuated. A control logic in the circuit may be configured to activate the electrodes in jaws (712, 714) with RF energy once trigger assembly (760) is fully actuated. It should be understood that the configuration of trigger assembly (760), catch (758), and projection (709) will prevent firing beam (750) from advancing distally until after the electrodes in jaws (712, 714) have been activated with RF energy.

In an exemplary use, cartridge portion (702) and grip portion (704) are initially provided as separate components. Body (708) of cartridge portion (702) is then inserted into grip portion (704) until latch (706) snaps into place to secure portions (702, 704) together. At this stage, contacts (701) engage contacts in grip portion (704) to provide a path for electrical continuity between portions (702, 704); and magnet (782) cooperates with reed switch (772) to register the coupling of portions (702, 704). Jaws (712, 714) are then positioned at a surgical site in a patient, with tissue between jaws (712, 714). The operator then squeezes rings (724, 734) toward each other to compress the tissue between jaws (712, 714). Once links (744, 740) reach a point where pin (748) enters notch (759) of firing beam (750) and catch (758) bears against protrusion (709), arm (720) can pivot no further toward arm (730). The operator then pivots trigger assembly (760) relative to second arm (730). Magnet (767) eventually trips reed switch (776), which then causes RF energy to be delivered to electrodes in jaws (712,714). In addition, chamfer (765) cams against catch (758), deflecting catch (758) out of engagement with protrusion (709). The operator then squeezes rings (724, 734) further, causing links (740, 744) to pivot to generally straight positions, thereby driving firing beam (750) distally. It should be understood that first arm (720) may bend to some degree during this stage. The distally advancing firing beam (750) severs the tissue between jaws (712, 714). Once firing beam (750) reaches the distal position, magnet (784) trips reed switch (774), effectively cutting off the RF energy at the electrodes in jaws (712, 714). The operator then relaxes their grip on rings (724, 734), releasing the tissue from jaws (712, 714) and retracting firing beam (750) proximally.

The above process may be repeated as many times as desired. For instance, jaws (712, 714) and firing beam (750) may be actuated repeatedly along a continuous line for any suitable length. Alternatively, jaws (712, 714) and firing beam (750) may be actuated repeatedly at different tissue sites. It should also be understood that jaws (712, 714) may be used to only grasp tissue, or to only grasp and seal tissue, without necessarily also severing the tissue with firing beam (750). After the operator is done using instrument (700), the operator may depress latch (706) and separate cartridge portion (702) from grip portion (704). The operator may then dispose of cartridge portion (702) and send grip portion (704) through a sterilization/reclamation process. Grip portion (704) may thus be later used in another surgical procedure with another cartridge portion (702). Other suitable components, features, variations, and operabilities for instrument (700) will be apparent to those of ordinary skill in the art in view of the teachings herein.

### III. Exemplary Electrosurgical Forceps with Firing Beam Slider

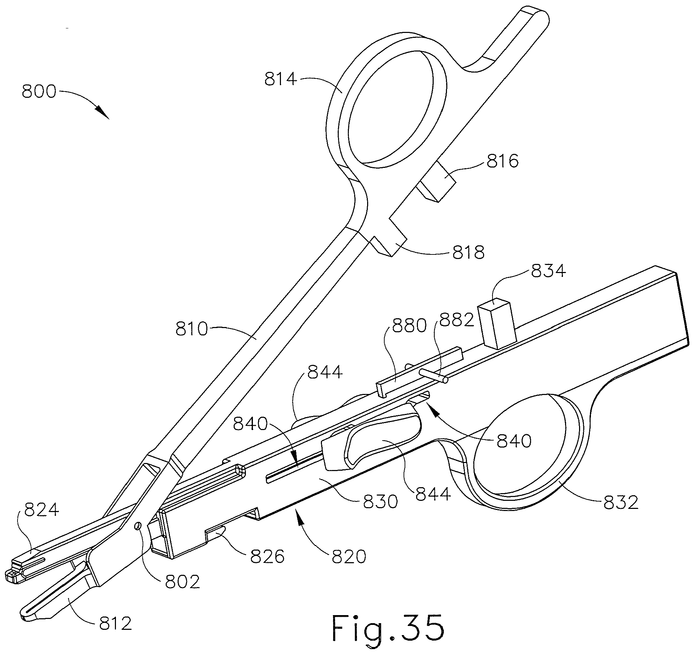

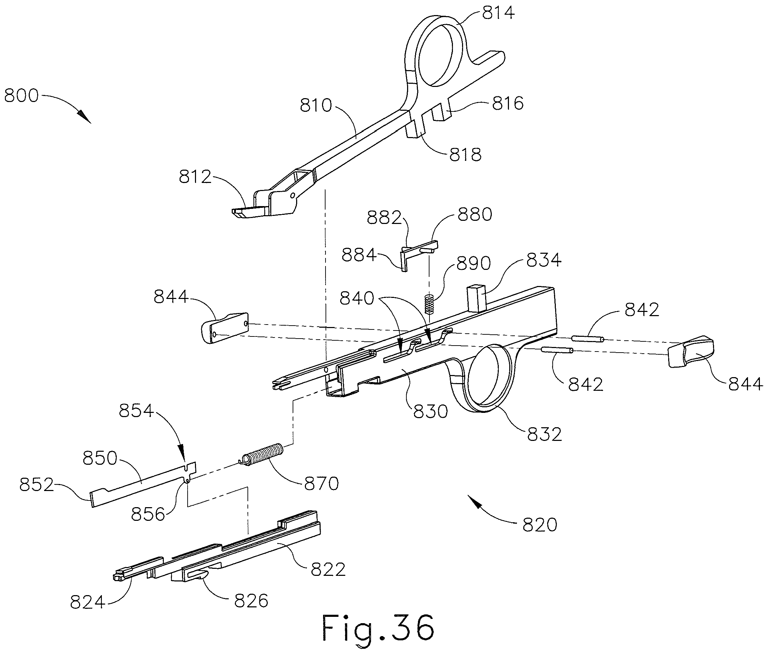

FIGS. 35-37 show yet another exemplary electrosurgical instrument (800) that may be used to grasp tissue, seal tissue, and sever tissue. Instrument (800) of this example includes a first arm (810) and a second arm assembly (820) that are pivotally coupled by a pin (802). A first jaw (812) is positioned at the distal end of first arm (810); while a thumb ring (814) is positioned at the proximal end of first arm (810). Second arm assembly (820) includes a cartridge body (822) and a grip housing (830). A second jaw (824) is positioned at the distal end of cartridge body (822). Cartridge body (822) also includes a resilient latch (826) that is configured to releasably couple cartridge body (822) with grip housing (830). Grip housing (830) includes a finger ring (832). First arm (810) and grip housing (830) comprise complementary ratcheting features (816, 834) that are configured to engage each other as first arm (810) pivots toward grip housing (830), thereby selectively locking the pivotal position of first arm (810) relative to grip housing (830). Ratcheting features (816, 834) may be configured similar to ratcheting features on conventional forceps instruments. Of course, ratcheting features (816) are merely optional and may be omitted if desired. Jaws (812, 822) include electrodes (not shown) that are similar to electrodes (113, 123) described above. Instrument (800) may also include a cable coupled with a control unit and power source, similar to cable (130), control unit (132), and power source (134) described above.

Grip housing (830) also includes a pair of dogleg slots (840). Dogleg slots (840) each include an upper longitudinally extending portion, a lower longitudinally extending portion, and a slanted portion coupling the upper and lower longitudinally extending portions. A pair of transversely oriented pins (842) are slidably positioned in slots (840), each pin (842) being located in a respective slot (840). The ends of pins (842) are secured to slider actuators (844), which are positioned lateral to grip housing (830). Actuators (844) are operable to slide pins (842) along the length of slots (840). The distal-most pin (842) is configured to engage a notch (854) formed in a firing beam (850). Firing beam (850) of this example includes a distal cutting edge (852) and is configured to translate distally through jaws (812, 824) to sever tissue captured between jaws (812, 824). Firing beam (850) also includes a lower projection (856) that is coupled with one end of a coil spring (870). The other end of coil spring (870) is secured to grip housing (830). Coil spring (870) is configured to resiliently bias firing beam (850) toward a proximal position, retracted proximal to jaws (812, 824).

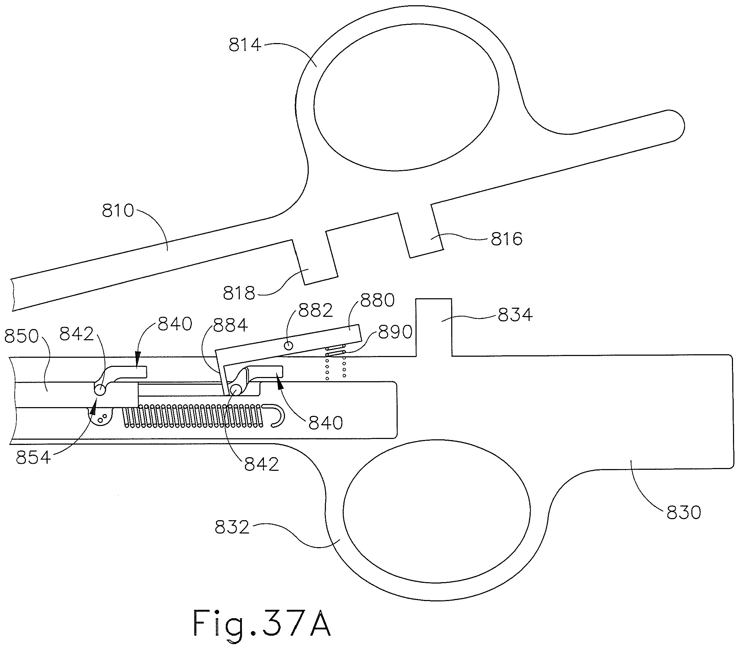

As can be seen from FIGS. 37A-37B, the dogleg configuration of slots (840) allows the distal-most pin (842) to selectively engage and disengage notch (854) of firing beam (850). This selective engagement may be performed when the operator wishes to couple or exchange cartridge bodies (822). For instance, when an operator wishes to initially couple a cartridge body (822) with grip housing (830), the operator may retract slider actuators (844) fully proximally, such that pins (842) are positioned in the upper longitudinally extending portions of respective slots (840). This may provide sufficient clearance for the proximal end of firing beam (850) to be fully seated relative to grip housing (830). Once latch (826) has sufficiently coupled with grip housing (830), the operator may slide slider actuators (844) distally to transition pins (842) along the slanted portions of slots (840) and down into the proximal ends of the lower longitudinally extending portions of respective slots. This positions the distal-most pin (842) in notch (854) of firing beam (850), as shown in FIG. 37A.

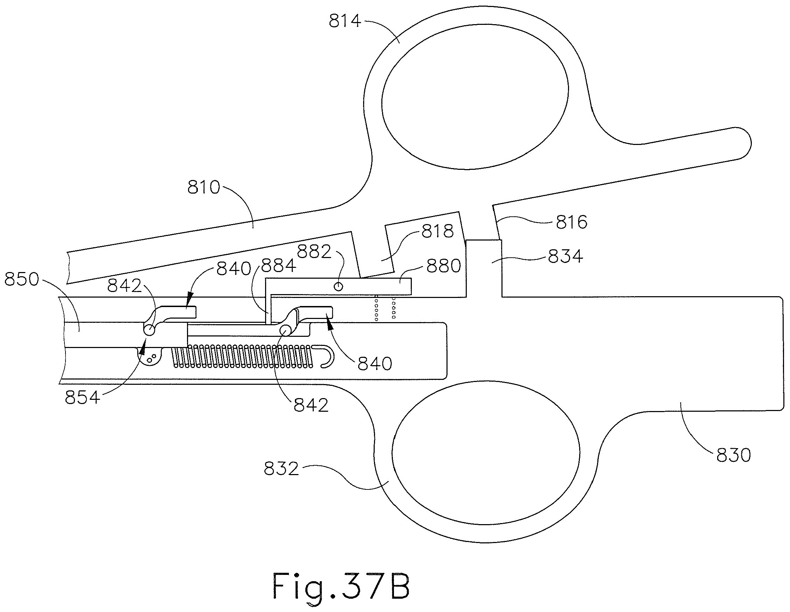

Once pins (842) reach the position shown in FIG. 37A, a blocking projection (884) prevents the distal-most pin (842) from moving further distally. Projection (884) projects downwardly from a pivot arm (880), which is pivotally coupled with grip housing (830) by a pin (882). A coil spring (890) resiliently biases pivot arm (880) to the position shown in FIG. 37A. It should be understood that, by preventing further distal movement of pins (842), blocking projection (884) prevents firing beam (850) from being advanced distally through jaws (812, 824). As first arm (810) is pivoted toward grip housing (830), a downwardly projecting member (818) of first arm (810) eventually engages pivot arm (880) and pivots arm (880) about pin (882) to the position shown in FIG. 37B. This moves projection (884) out of the path of the distal-most pin (842), and thus allows slider actuators (844) to be slid further distally to drive firing beam (850) through jaws (812, 824). Jaws (812, 824) are closed by the time projecting member (818) pivots arm (880) to the position shown in FIG. 37B. It should be understood from the foregoing that pivot arm (880) and projection (884) prevent firing beam (850) from being advanced distally through jaws (812, 824) until jaws (812, 824) are closed.

In an exemplary use, cartridge body (822) and the rest of instrument (800) are initially provided as separate components. Cartridge body (822) is then inserted into grip housing (830) until latch (826) snaps into place to secure body (822) and housing (830) together. Jaws (812, 824) are then positioned at a surgical site in a patient, with tissue between jaws (812, 824). The operator then squeezes rings (814, 832) toward each other to compress the tissue between jaws (812, 824). Downwardly projecting member (818) engages arm (880) and pivots arm (880) about pin (882), from the position shown in FIG. 37A to the position shown in FIG. 37B. Any suitable type of activation feature may be activated to provide RF energy at the electrodes in jaws (812, 824), to thereby seal the tissue captured between jaws (812, 824). The operator then advances slider actuators (844) distally to advance firing beam (850) distally, thereby severing tissue captured between jaws (812, 824). Once slider actuators (844) and firing beam (850) reach a distal-most position, the operator may release slider actuators (844), allowing spring (870) to return firing beam (850) and slider actuators (844) back to a proximal position.

The above process may be repeated as many times as desired. For instance, jaws (812, 824) and firing beam (850) may be actuated repeatedly along a continuous line for any suitable length. Alternatively, jaws (812, 824) and firing beam (850) may be actuated repeatedly at different tissue sites. It should also be understood that jaws (812, 824) may be used to only grasp tissue, or to only grasp and seal tissue, without necessarily also severing the tissue with firing beam (850). After the operator is done using instrument (800), the operator may depress latch (826) and separate cartridge body (822) from grip housing (830). The operator may then dispose of cartridge body (822) and send the rest of instrument (800) through a sterilization/reclamation process. The rest of instrument (800) may thus be later used in another surgical procedure with another cartridge body (822). Other suitable components, features, variations, and operabilities for instrument (800) will be apparent to those of ordinary skill in the art in view of the teachings herein.

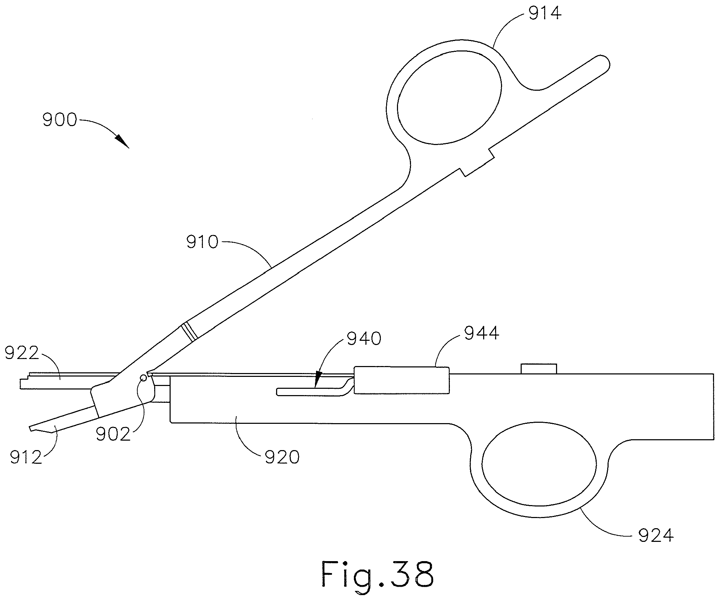

FIGS. 38-39F show yet another exemplary electrosurgical instrument (900) that may be used to grasp tissue, seal tissue, and sever tissue. Instrument (900) of this example includes a first arm (910) and a second arm (920) that are pivotally coupled by a pin (902). A first jaw (912) is positioned at the distal end of first arm (910); while a thumb ring (914) is positioned at the proximal end of first arm (910). A second jaw (922) is positioned at the distal end of second arm (920); while a thumb ring (924) is positioned at the proximal end of first arm (910). Jaws (912, 922) include electrodes (not shown) that are similar to electrodes (113, 123) described above. Instrument (900) may also include a cable coupled with a control unit and power source, similar to cable (130), control unit (132), and power source (134) described above.

Second arm (920) also includes a pair of dogleg slots (940). Dogleg slots (940) each include an upper longitudinally extending portion, a lower longitudinally extending portion, and a slanted portion coupling the upper and lower longitudinally extending portions. A pair of transversely oriented pins (942) are slidably positioned in slots (940), each pin (942) being located in a respective slot (940). The ends of pins (942) are secured to slider actuators (944), which are positioned lateral to second arm (920). Actuators (944) are operable to slide pins (942) along the length of slots (940). The distal-most pin (942) is configured to engage a notch (954) formed in a firing beam (950). Firing beam (950) of this example includes a distal cutting edge (not shown) and is configured to translate distally through jaws (912, 922) to sever tissue captured between jaws (912, 922). One end of a coil spring (970) is secured to slider actuators (944) while the other end of coil spring (970) is secured to second arm (920). Coil spring (970) is configured to resiliently bias slider actuators (944) toward a proximal position.

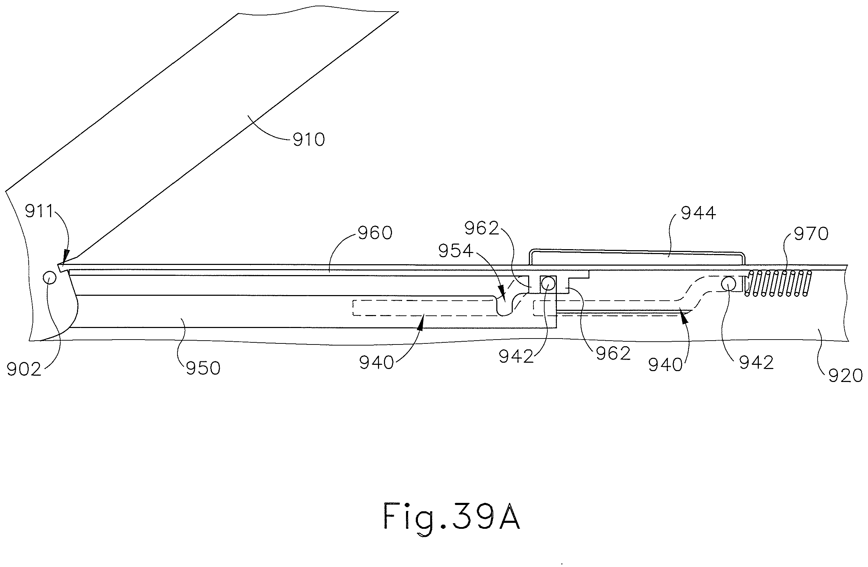

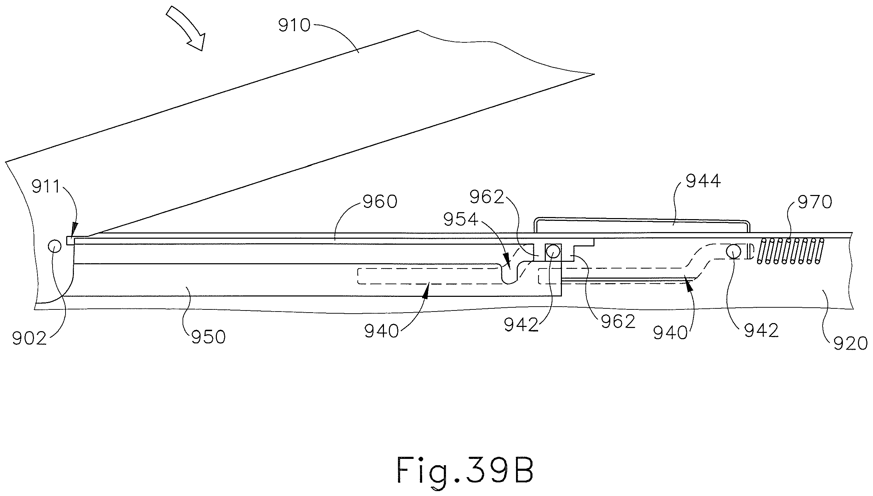

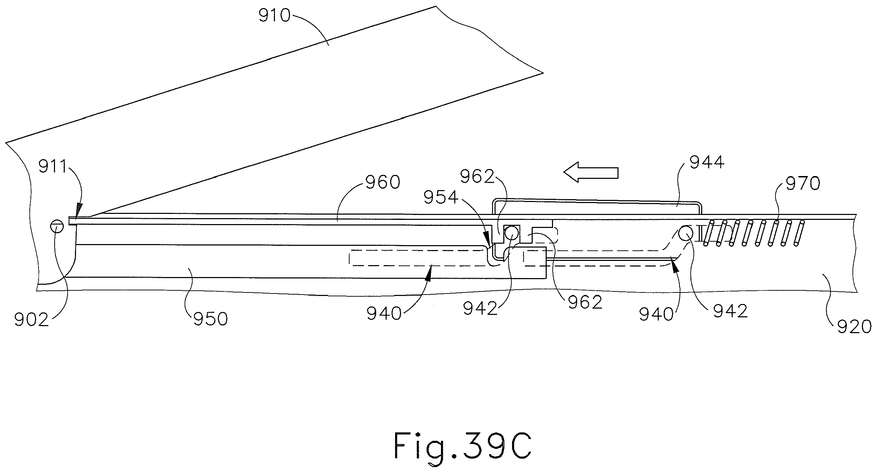

As shown in FIGS. 39A-39F, a jaw lock beam (960) is slidably disposed in second arm (920). The distal end of jaw lock beam (960) is configured to engage a notch (911) formed in first arm (910). A pair of prongs (962) extend transversely from lock beam (960) and couple lock beam (960) with the distal-most pin (942). As shown in FIG. 39A, the distal end of jaw lock beam (960) is below and proximal to notch (911) when first arm (910) is pivoted to an open position, where jaws (912, 922) are open. However, when first arm (910) is pivoted to a closed position, where jaws (912, 922) are closed, notch (911) is aligned with the distal end of jaw lock beam (960) as shown in FIG. 39B. The operator may then slide actuators (944) distally, driving the distal end of jaw lock beam (960) into notch (911) as shown in FIG. 39C. With the distal end of jaw lock beam (960) disposed in notch (911), first arm (910) cannot be pivoted away from second arm (920). Jaws (912, 922) are thus effectively locked in the closed position. Any suitable type of activation feature may be activated to provide RF energy at the electrodes in jaws (912, 922), to thereby seal the tissue captured between jaws (912, 922).

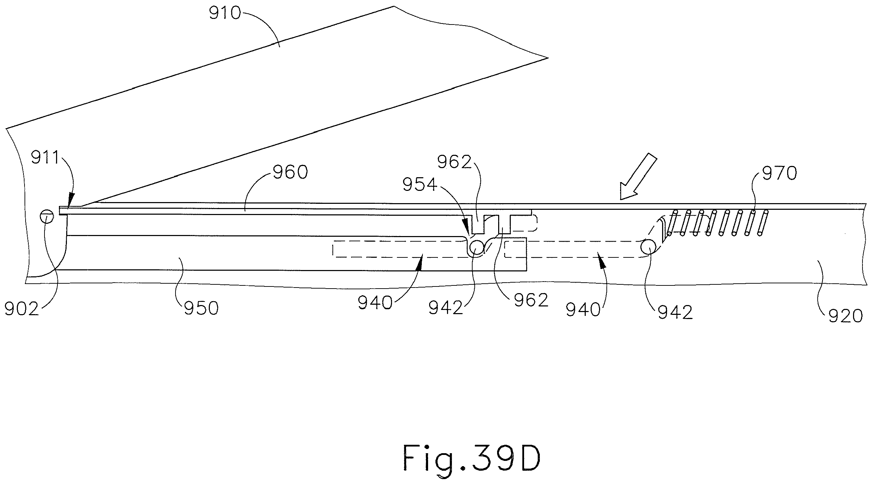

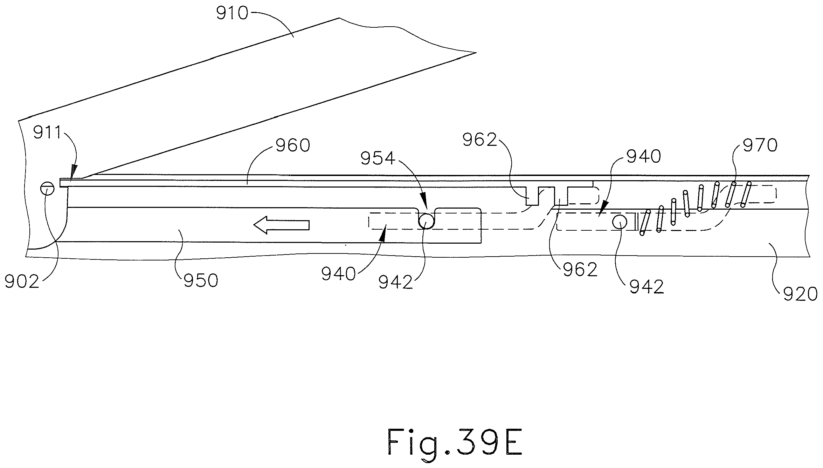

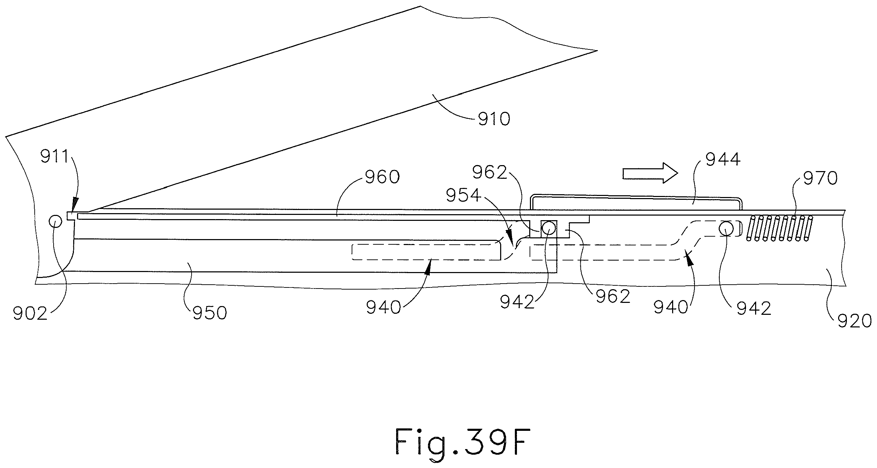

As the operator continues to advance actuators (944) distally, pins (942) transition along the slanted portions coupling the upper and lower longitudinally extending portions of slots (840), such that the distal-most pin (942) disengages prongs (962) as shown in FIG. 39D. The distal end of jaw lock beam (960) nevertheless remains disposed in notch (911). The operator may continue to advance actuators (944) distally as shown in FIG. 39E, driving firing beam (950) distally to sever tissue captured between jaws (912, 922). The operator may then release actuators (944), allowing spring (970) to pull actuators (944) and firing beam (950) back proximally to the position shown in FIG. 39F. During this transit, the distal-most pin (942) re-engages prongs (962) and pulls jaw lock beam (960) proximally, such that the distal end of jaw lock beam (960) disengages notch (911) of first arm (910), thereby allowing arm (910) and jaw (912) to be pivoted once again.

The above process may be repeated as many times as desired. For instance, jaws (912, 922) and firing beam (950) may be actuated repeatedly along a continuous line for any suitable length. Alternatively, jaws (912, 922) and firing beam (950) may be actuated repeatedly at different tissue sites. It should also be understood that jaws (912, 922) may be used to only grasp tissue, or to only grasp and seal tissue, without necessarily also severing the tissue with firing beam (950). Other suitable components, features, variations, and operabilities for instrument (900) will be apparent to those of ordinary skill in the art in view of the teachings herein.

### IV. Miscellaneous

It should be understood that any of the versions of instruments described herein may include various other features in addition to or in lieu of those described above. By way of example only, any of the instruments described herein may also include one or more of the various features disclosed in any of the various references. It should also be understood that the teachings herein may be readily applied to any of the instruments described in any of the other references cited herein, such that the teachings herein may be readily combined with the teachings of any of the references cited herein in numerous ways. Other types of instruments into which the teachings herein may be incorporated will be apparent to those of ordinary skill in the art.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI™ system by Intuitive Surgical, Inc., of Sunnyvale, California. Similarly, those of ordinary skill in the art will recognize that various teachings herein may be readily combined with various teachings of U.S. Pat. No. 6,783,524, entitled "Robotic Surgical Tool with Ultrasound Cauterizing and Cutting Instrument," published August 31, 2004.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus (100) for operating on tissue, the apparatus (100) comprising:
(a) a first arm (110), wherein the first arm (110) comprises a first jaw (112), wherein the first jaw (112) includes an electrode (113) operable to deliver RF energy to tissue;
(b) a second arm (120), wherein the second arm (120) comprises a second jaw (122), wherein the second jaw (122) includes an electrode (123) operable to deliver RF energy to tissue, wherein the first arm (110) is pivotable relative to the second arm (120);
(c) a firing beam (150), wherein the firing beam (150) is operable to translate distally through the first and second jaws (112, 122) to sever tissue captured between the first and second jaws (112, 122);
(d) a first link (140) pivotably coupled with the first arm (110), wherein the first link (140) is further pivotably coupled with the firing beam (150), wherein the first link (140) is operable to advance the firing beam (150) distally in response to pivotal movement of the first arm (110) toward the second arm (120);
(e) a lockout feature operable to selectively prevent translation of the firing beam (150); and
(f) a scissor grip portion associated with the first and second arms (110, 120), wherein the scissor grip portion is operable to pivot the first arm (110) toward the second arm (120).

2. The apparatus (100) of claim 1, wherein the lockout feature is configured to selectively restrict pivoting of the first link (140).

3. The apparatus (100) of claim 1, wherein the first arm (110) is pivotable relative to the second arm (120) through a first range of motion to move the first jaw (112) from an open position to a closed position in relation to the second jaw (122) without advancing the firing beam (150) distally.

4. The apparatus (100) of claim 3, wherein the first arm (110) is pivotable relative to the second arm (120) through a second range of motion to advance the firing beam (150) distally while the first jaw (112) remains in a closed position in relation to the second jaw (122).

5. The apparatus (100) of claim 4, wherein the first arm (110) is configured to deform during the second range of motion.

6. The apparatus (700) of claim 1, further comprising a second link (744) pivotably coupled with the first arm (720), wherein the second link (744) is further pivotably coupled with the firing beam (750) such that the second link (744) provides a pivoting coupling between the first link (740) and the firing beam (750).

7. The apparatus (100) of claim 1, wherein the first link (140) is coupled with the first arm (110) by a pivot joint, wherein the pivot joint includes a laterally projecting feature (144), wherein the second arm includes a channel (128) configured to receive the laterally projecting feature.

8. The apparatus (100) of claim 1, further comprising a trigger (136) operable to activate RF energy at the electrodes (113, 123) of the first and second jaws (110, 120).

9. The apparatus (100) of claim 8, wherein the trigger (360) is further operable to engage the lockout feature (370) to enable translation of the firing beam (350).

10. The apparatus (100) of claim 8, wherein the trigger (360) is configured to engage the lockout feature (370) to enable translation of the firing beam (350) after or contemporaneously with activating RF energy at the electrodes of the first and second jaws (312, 322).

11. The apparatus (100) of claim 10, wherein the trigger (360) is movable through a first range of motion to activate RF energy at the electrodes of the first and second jaws (312, 322) without engaging the lockout feature to enable translation of the firing beam.

12. The apparatus (100) of claim 11, wherein the trigger (360) is movable through a second range of motion to engage the lockout feature (370) to enable translation of the firing beam (350) while still activating RF energy at the electrodes of the first and second jaws (312, 322).

13. The apparatus (100) of claim 8, wherein the firing beam (350) defines a notch (358), wherein the lockout feature (370) comprises a pivoting member operable to selectively engage the notch (358) of the firing beam (350) to selectively prevent translation of the firing beam (350), wherein the trigger (360) is operable to pivot the pivoting member to disengage the pivoting member from the notch (358) of the firing beam.

14. The apparatus (600) of claim 8, wherein the firing beam (650) defines a notch (658), wherein the lockout feature comprises a resilient member (670) operable to selectively engage the notch (658) of the firing beam (650) to selectively prevent translation of the firing beam (650), wherein the trigger (660) is operable to deform the resilient member (670) to disengage the resilient member (670) from the notch (658) of the firing beam (650).

15. The apparatus of claim 8, wherein the firing beam defines a catch feature (758), wherein the lockout feature comprises a protrusion operable to selectively engage the notch of the firing beam (750) to selectively prevent translation of the firing beam (750), wherein the trigger (760) is operable to deform the firing beam (750) to disengage the catch feature (758) from the protrusion (709).

16. The apparatus (600) of claim 1, further comprising:
(a) a cartridge assembly (602); and
(b) grip housing (604), wherein the cartridge assembly (602) is removably coupled with the grip housing (604) by a resilient latch feature (630).

17. The apparatus (600) of claim 16, wherein the cartridge assembly (602) comprises:
(i) the first jaw (612),
(ii) the second jaw (622), and
(iii) the first arm (610).

18. The apparatus of claim 16, wherein the scissor grip portion comprises:
(i) a first ring (614) positioned on the first arm (610), and
(ii) a second ring (624) positioned on the grip housing (602).

## Patentansprüche

1. Vorrichtung (100) für Gewebeoperationen, wobei die Vorrichtung (100) Folgendes umfasst:
(a) einen ersten Arm (110), wobei der erste Arm (110) eine erste Backe (112) umfasst, wobei die erste Backe (112) eine Elektrode (113) aufweist, die dahingehend betätigbar ist, HF-Energie an Gewebe zu liefern,
(b) einen zweiten Arm (120), wobei der zweite Arm (120) eine zweite Backe (122) umfasst, wobei die zweite Backe (122) eine Elektrode (123) aufweist, die dahingehend betätigbar ist, HF-Energie an Gewebe zu liefern, wobei der erste Arm (110) bezüglich des zweiten Arms (120) schwenkbar ist,
(c) einen Auslösebalken (150), wobei der Auslösebalken (150) dahingehend betätigbar ist, distal durch die erste und die zweite Backe (112, 122) zu translatieren, um zwischen der ersten und der zweiten Backe (112, 122) eingeklemmtes Gewebe abzutrennen,
(d) ein erstes Verbindungsglied (140), das schwenkbar mit dem ersten Arm (110) gekoppelt ist, wobei das erste Verbindungsglied (140) ferner schwenkbar mit dem Auslösebalken (150) gekoppelt ist, wobei das erste Verbindungsglied (140) dahingehend betätigbar ist, den Auslösebalken (150) als Reaktion auf eine Schwenkbewegung des ersten Arms (110) zum zweiten Arm (120) hin distal vorzuschieben,
(e) ein Sperrmerkmal, das dahingehend betätigbar ist, eine Translation des Auslösebalkens (150) gezielt zu verhindern, und
(f) einen Scherengriffabschnitt, der dem ersten und dem zweiten Arm (110, 120) zugeordnet ist, wobei der Scherengriffabschnitt dahingehend betätigbar ist, den ersten Arm (110) zum zweiten Arm (120) hin zu schwenken.

2. Vorrichtung (100) nach Anspruch 1, wobei das Sperrmerkmal dazu konfiguriert ist, ein Schwenken des ersten Verbindungsglieds (140) gezielt zu beschränken.

3. Vorrichtung (100) nach Anspruch 1, wobei der erste Arm (110) durch einen ersten Bewegungsbereich bezüglich des zweiten Arms (120) schwenkbar ist, um die erste Backe (112) aus einer offenen Position in eine geschlossene Position bezüglich der zweiten Backe (122) zu bewegen, ohne den Auslösebalken (150) distal vorzuschieben.

4. Vorrichtung (100) nach Anspruch 3, wobei der erste Arm (110) durch einen zweiten Bewegungsbereich bezüglich des zweiten Arms (120) schwenkbar ist, um den Auslösebalken (150) distal vorzuschieben, während die erste Backe (112) in einer geschlossenen Position bezüglich der zweiten Backe (122) bleibt.

5. Vorrichtung (100) nach Anspruch 4, wobei der erste Arm (110) dazu konfiguriert ist, sich während des zweiten Bewegungsbereichs zu deformieren.

6. Vorrichtung (700) nach Anspruch 1, ferner umfassend ein zweites Verbindungsglied (744), das schwenkend mit dem ersten Arm (720) gekoppelt ist, wobei das zweite Verbindungsglied (744) ferner schwenkend mit dem Auslösebalken (750) gekoppelt ist, so dass das zweite Verbindungsglied (744) eine Schwenkkoppelung zwischen dem ersten Verbindungsglied (740) und dem Auslösebalken (750) bereitstellt.

7. Vorrichtung (100) nach Anspruch 1, wobei das erste Verbindungsglied (140) über ein Schwenkgelenk mit dem ersten Arm (110) gekoppelt ist, wobei das Schwenkgelenk ein seitlich vorragendes Merkmal (144) aufweist, wobei der zweite Arm einen Kanal (128) aufweist, der zur Aufnahme des seitlich vorragenden Merkmals konfiguriert ist.

8. Vorrichtung (100) nach Anspruch 1, ferner umfassend einen Auslöser (136), der dahingehend betätigbar ist, HF-Energie an den Elektroden (113, 123) der ersten und der zweiten Backe (110, 120) zu aktivieren.

9. Vorrichtung (100) nach Anspruch 8, wobei der Auslöser (360) ferner dahingehend betätigbar ist, das Sperrmerkmal (370) in Eingriff zu nehmen, um eine Translation des Auslösebalkens (350) zu ermöglichen.

10. Vorrichtung (100) nach Anspruch 8, wobei der Auslöser (360) dazu konfiguriert ist, das Sperrmerkmal (370) in Eingriff zu nehmen, um eine Translation des Auslösebalkens (350) nach der Aktivierung von HF-Energie an den Elektroden der ersten und der zweiten Backe (312, 322) oder gleichzeitig damit zu ermöglichen.

11. Vorrichtung (100) nach Anspruch 10, wobei der Auslöser (360) durch einen ersten Bewegungsbereich bewegbar ist, um HF-Energie an den Elektroden der ersten und der zweiten Backe (312, 322) zu aktivieren, ohne das Sperrmerkmal zur Ermöglichung einer Translation des Auslösebalkens in Eingriff zu nehmen.

12. Vorrichtung (100) nach Anspruch 11, wobei der Auslöser (360) durch einen zweiten Bewegungsbereich bewegbar ist, um das Sperrmerkmal (370) zur Ermöglichung einer Translation des Auslösebalkens (350) in Eingriff zu nehmen, während trotzdem HF-Energie an den Elektroden der ersten und der zweiten Backe (312, 322) aktiviert wird.

13. Vorrichtung (100) nach Anspruch 8, wobei der Auslösebalken (350) eine Kerbe (358) definiert, wobei das Sperrmerkmal (370) ein Schwenkglied umfasst, das dahingehend betätigbar ist, die Kerbe (358) des Auslösebalkens (350) gezielt in Eingriff zu nehmen, um eine Translation des Auslösebalkens (350) gezielt zu verhindern, wobei der Auslöser (360) dahingehend betätigbar ist, das Schwenkglied zu schwenken, um das Schwenkglied aus der Kerbe (358) des Auslösebalkens auszurücken.

14. Vorrichtung (600) nach Anspruch 8, wobei der Auslösebalken (650) eine Kerbe (658) definiert, wobei das Sperrmerkmal ein federndes Glied (670) umfasst, das dahingehend betätigbar ist, die Kerbe (658) des Auslösebalkens (650) gezielt in Eingriff zu nehmen, um eine Translation des Auslösebalkens (650) gezielt zu verhindern, wobei der Auslöser (660) dahingehend betätigbar ist, das federnde Glied (670) zu deformieren, um das federnde Glied (670) aus der Kerbe (658) des Auslösebalkens (650) auszurücken.

15. Vorrichtung nach Anspruch 8, wobei der Auslösebalken ein Rastmerkmal (758) definiert, wobei das Sperrmerkmal einen Vorsprung umfasst, der dahingehend betätigbar ist, die Kerbe des Auslösebalkens (750) gezielt in Eingriff zu nehmen, um eine Translation des Auslösebalkens (750) gezielt zu verhindern, wobei der Auslöser (760) dahingehend betätigbar ist, den Auslösebalken (750) zu deformieren, um das Rastmerkmal (758) aus dem Vorsprung (709) auszurücken.

16. Vorrichtung (600) nach Anspruch 1, ferner umfassend:
(a) eine Kartuschenanordnung (602) und
(b) ein Griffgehäuse (604), wobei die Kartuschenanordnung (602) über ein federndes Klinkenmerkmal (630) entfernbar mit dem Griffgehäuse (604) gekoppelt ist.

17. Vorrichtung (600) nach Anspruch 16, wobei die Kartuschenanordnung (602) Folgendes umfasst:
(i) die erste Backe (612),
(ii) die zweite Backe (622) und
(iii) den ersten Arm (610).

18. Vorrichtung nach Anspruch 16, wobei der Scherengriffabschnitt Folgendes umfasst:
(i) einen ersten Ring (614), der am ersten Arm (610) positioniert ist, und
(ii) einen zweiten Ring (624), der am Griffgehäuse (602) positioniert ist.

## Revendications

1. Appareil (100) servant à opérer du tissu, l'appareil (100) comprenant :
(a) un premier bras (110), le premier bras (110) comprenant une première mâchoire (112) ; la première mâchoire (112) comprenant une électrode (113) pouvant être mise en oeuvre pour délivrer de l'énergie RF au tissu ;
(b) un second bras (120), le second bras (120) comprenant une seconde mâchoire (122), la seconde mâchoire (122) comprenant une électrode (123) pouvant être mise en oeuvre pour délivrer de l'énergie RF au tissu, le premier bras (110) pouvant pivoter par rapport au second bras (120) ;
(c) une barre de projection (150), la barre de projection (150) pouvant être mise en oeuvre pour translater dans la direction distale à travers les première et seconde mâchoires (112, 122) de façon à sectionner du tissu en prise entre les première et seconde mâchoires (112, 122) ;
(d) un premier élément de liaison (140) accouplé à pivotement avec le premier bras (110), le premier élément de liaison (140) étant en outre accouplé à pivotement avec la barre de projection (150), le premier élément de liaison (140) pouvant être mis en oeuvre pour avancer la barre de projection (150) dans la direction distale en réaction à un mouvement de pivotement du premier bras (110) en direction du second bras (120) ;
(e) un élément de blocage pouvant être mis en oeuvre pour empêcher, de manière sélective, une translation de la barre de projection (150) ; et
(f) une partie de préhension de type ciseaux associée aux premier et second bras (110, 120), la partie de préhension de type ciseaux pouvant être mise en oeuvre pour faire pivoter le premier bras (110) en direction du second bras (120).

2. Appareil (100) selon la revendication 1, dans lequel l'élément de blocage est configuré pour limiter, de manière sélective, le pivotement du premier élément de liaison (140).

3. Appareil (100) selon la revendication 1, dans lequel le premier bras (110) peut pivoter par rapport au second bras (120) sur une première plage de mouvement afin de déplacer la première mâchoire (112) d'une position ouverte à une position fermée par rapport à la seconde mâchoire (122) sans avancer la barre de projection (150) dans la direction distale.

4. Appareil (100) selon la revendication 3, dans lequel le premier bras (110) peut pivoter par rapport au second bras (120) sur une seconde plage de mouvement afin d'avancer la barre de projection (150) dans la direction distale tandis que la première mâchoire (112) reste dans une position fermée par rapport à la seconde mâchoire (122).

5. Appareil (100) selon la revendication 4, dans lequel le premier bras (110) est configuré pour se déformer au sein de la seconde plage de mouvement.

6. Appareil (700) selon la revendication 1, comprenant en outre un second élément de liaison (744) accouplé à pivotement avec le premier bras (720), dans lequel le second élément de liaison (744) est accouplé en outre à pivotement avec la barre de projection (750) de telle sorte que le second élément de liaison (744) forme un accouplement pivotant entre le premier élément de liaison (740) et la barre de projection (750).

7. Appareil (100) selon la revendication 1, dans lequel le premier élément de liaison (140) est accouplé avec le premier bras (110) par un raccord pivotant, dans lequel le raccord pivotant comprend un élément faisant saillie latéralement (144), dans lequel le second bras comprend un canal (128) configuré pour recevoir l'élément faisant saillie latéralement.

8. Appareil (100) selon la revendication 1, comprenant en outre un élément de déclenchement (136) pouvant être mis en oeuvre pour activer de l'énergie RF au niveau des électrodes (113, 123) des première et seconde mâchoires (110, 120).

9. Appareil (100) selon la revendication 8, dans lequel l'élément de déclenchement (360) peut être mis en oeuvre en outre pour interagir avec l'élément de blocage (370) afin de permettre une translation de la barre de projection (350).

10. Appareil (100) selon la revendication 8, dans lequel l'élément de déclenchement (360) est configuré pour interagir avec l'élément de blocage (370) afin de permettre une translation de la barre de projection (350) après l'activation d'énergie RF au niveau des électrodes des première et seconde mâchoires (312, 322) ou simultanément avec celle-ci.

11. Appareil (100) selon la revendication 10, dans lequel l'élément de déclenchement (360) est mobile sur une première plage de mouvement afin d'activer de l'énergie RF au niveau des électrodes des première et seconde mâchoires (312, 322) sans interagir avec l'élément de blocage afin de permettre une translation de la barre de projection.

12. Appareil (100) selon la revendication 11, dans lequel l'élément de déclenchement (360) est mobile sur une seconde plage de mouvement afin d'interagir avec l'élément de blocage (370) afin de permettre une translation de la barre de projection (350) tout en continuant d'activer de l'énergie RF au niveau des électrodes des première et seconde mâchoires (312, 322).

13. Appareil (100) selon la revendication 8, dans lequel la barre de projection (350) définit un cran (358), dans lequel l'élément de blocage (370) comprend un organe pivotant pouvant être mis en oeuvre pour venir en prise, de manière sélective, avec le cran (358) de la barre de projection (350) afin d'empêcher, de manière sélective, une translation de la barre de projection (350), dans lequel l'élément de déclenchement (360) peut être mis en oeuvre pour faire pivoter l'organe pivotant afin de libérer l'organe pivotant du cran (358) de la barre de projection.

14. Appareil (600) selon la revendication 8, dans lequel la barre de projection (650) définit un cran (658), dans lequel l'élément de blocage comprend un organe élastique (670) pouvant être mis en oeuvre pour venir en prise, de manière sélective, avec le cran (658) de la barre de projection (650) afin d'empêcher, de manière sélective, une translation de la barre de projection (650), dans lequel l'élément de déclenchement (660) peut être mis en oeuvre pour déformer l'organe élastique (670) afin de libérer l'organe élastique (670) du cran (658) de la barre de projection (650).

15. Appareil selon la revendication 8, dans lequel la barre de projection définit un élément d'arrêt (758), dans lequel l'élément de blocage comprend une protubérance pouvant être mise en oeuvre pour venir en prise, de manière sélective, avec le cran de la barre de projection (750) afin d'empêcher, de manière sélective, une translation de la barre de projection (750), dans lequel l'élément de déclenchement (760) peut être mis en oeuvre pour déformer la barre de projection (750) afin de libérer l'élément d'arrêt (758) de la protubérance (709).

16. Appareil (600) selon la revendication 1, comprenant en outre :
(a) un ensemble formant cartouche (602) ; et
(b) un logement formant élément de préhension (604), l'ensemble formant cartouche (602) étant accouplé, de manière amovible, avec le logement formant élément de préhension (604) par le biais d'un élément de verrouillage élastique (630).

17. Appareil (600) selon la revendication 16, dans lequel l'ensemble formant cartouche (602) comprend :
(i) la première mâchoire (612),
(ii) la seconde mâchoire (622), et
(iii) le premier bras (610).

18. Appareil selon la revendication 16, dans lequel la partie de préhension de type ciseaux comprend :
(i) un premier anneau (614) positionné sur le premier bras (610), et
(ii) un second anneau (624) positionné sur le logement formant élément de préhension (602).
